# EUROPEAN PATENT APPLICATION

(11) **EP 3 422 316 A1**
(43) Date of publication of application: **02.01.2019**
(21) Application number: 17756450.7
(22) Date of filing: 21.02.2017
(51) Int. Cl.: G08B 25/00, A61G 12/00, G08B 13/196, G08B 25/04, H04N 7/18

(54) **MONITORED-PERSON MONITORING DEVICE, METHOD AND SYSTEM**

(30) Priority: 24.02.2016 JP 2016032905
(71) Applicant: Konica Minolta, Inc., Tokyo 100-7015 (JP)
(72) Inventor: NISHIKADO, Masashi, Tokyo 100-7015 (JP); HORIE, Daisaku, Tokyo 100-7015 (JP); KOYANAGI, Hitoshi, Tokyo 100-7015 (JP); FUJIWARA, Koji, Tokyo 100-7015 (JP); NODA, Atsuhiro, Tokyo 100-7015 (JP)
(74) Representative: Burton, Nick
(86) International application number: PCT/JP2017/006248
(87) International publication number: WO 2017/146012

(57) **Abstract**

Monitoring target person monitoring device, method, and system according to the present invention sense a predetermined event regarding a monitoring target person to notify the event; acquire an image including at least a video; determine, based on the acquired image, whether or not multiple persons are on the image; and start, in a case where it is determined that the multiple persons are on the image, storing the acquired video to store the video in a video storage.

## Description

### Technical Field

The present invention relates to a monitoring target person monitoring device, a monitoring target person monitoring method, and a monitoring target person monitoring system for monitoring a monitoring target person as a monitoring target to be monitored.

### Background Art

Due to, e.g., improvement in living standards, improvement in hygienic environment, and improvement in medical standards in association with rapid economic growth after the war, our country (Japan) is on the way to an aging society, more specifically a super-aging society in which a population aging rate as the percentage of the population aged 65 and over with respect to the total population exceeds 21%. In 2005, the elderly population aged 65 or over with respect to a total population of about 127,650,000 is about 25,560,000. On the other hand, it is expected that in 2020, the elderly population with respect to a total population of about 124,110,000 is about 34,560,000. In this aging society, it is expected that care-requiring persons or care receivers (care-requiring persons etc.) requiring nursing care or elderly care due to diseases, injuries, aging, etc. increase as compared to care-requiring persons etc. in a normal society which is not the aging society. Moreover, our country is also in a society with fewer children, and in this society, a total fertility rate in 2013 is 1.43, for example. For these reasons, an elderly-care-by-the-elderly situation has occurred, in which an elderly family member (a marital partner, a child, a brother or sister) provides elderly care to an elderly care-requiring person etc.

The care-requiring persons etc. are moved into facilities such as hospitals and welfare facilities for the elderly (under the Japanese laws, respite care facilities for the elderly, elderly nursing homes, and special elderly nursing homes), and receive nursing care or elderly care. In these facilities, a situation might occur, in which the care-requiring persons etc. get injured due to, e.g., falling from beds or overturning during walking or sneak out of beds to wander around. Countermeasures against such a situation need to be taken as quickly as possible. If such a situation is left unsolved, this might lead to a more serious situation. For these reasons, in the above-described facilities, nurses, caregivers, etc. regularly patrol to check the safety and state of the care-requiring persons etc.

However, the increasing number of nurses etc. cannot follow the increasing number of care-requiring persons etc., and therefore, a nursing care or elderly care industry has been short-handed chronically. Further, the number of nurses, caregivers, etc. is less in a time period for an evening shift or a night shift than in a time period for a day shift. This leads to an increase in a burden in job per person, and for this reason, reduction in the burden in job has been demanded. The above-described elderly-care-by-the-elderly situation occurs in the above-described facilities without exception, and a situation where elderly nurses etc. provide care to elderly care-requiring persons etc. is frequently encountered. Generally, body strength is reduced as one grows older. For this reason, even if the elderly nurses etc. have normal health, a burden on the elderly nurses etc. is greater than that on young nurses etc., and motion and judgement of the elderly nurses etc. are slower.

For reducing short-handedness and the burden on the nurses etc., the technique of complementing a nursing care service or an elderly care service has been demanded. Thus, in recent years, the monitoring target person monitoring techniques of monitoring (providing monitoring) a monitoring target person as a monitoring target to be monitored, such as the care-requiring persons etc., have been studied and developed.

For example, one of these techniques is a nurse call system disclosed in Patent Literature 1. The nurse call system disclosed in Patent Literature 1 has a nurse call child device placed at a bed to call a nurse by a patient, and a nurse call parent device placed at a nurses' station to reply to calling from the nurse call child device. The nurse call system further has a camera configured to capture an image of the patient on the bed from above, and a state determination section configured to determine, from a video captured by the camera, occurrence of at least one of a state in which the patient sits up or a state in which the patient left the bed to output an attentional state occurrence signal. The nurse call parent device has an informing section configured to perform informing operation in response to the attentional state occurrence signal. This nurse call system further has a mobile terminal carried by the nurse to reply to calling from the nurse call child device, and a communication control section configured to transmit the video captured by the camera to the mobile terminal in response to the attentional state occurrence signal.

In terms of safety checking, persons living on their own are also monitoring target persons as in the care-requiring persons etc.

As in the nurse call system disclosed in Patent Literature 1, when the video from the camera is displayed on the terminal device, a monitoring person such as a nurse can visually grasp the status of a monitoring target person such as a care-requiring person. Thus, this is convenient. Among monitoring target persons, some are less movable or bedridden. Thus, prevention of a theft etc. has been demanded. Moreover, it has been demanded to record a state in nursing care or assistance for a monitoring target person. In the nurse call system disclosed in Patent Literature 1, the video can be captured by the camera, and therefore, can be recorded. However, it is not preferable because a great storage capacity is necessary when the video is merely recorded. Moreover, it is not preferable because the privacy of a monitoring target person is not taken into consideration.

### Citation List

### Patent Literature

### Patent Literature 1: JP 2014-90913 A

### Summary of Invention

The present invention is an invention made in view of the above-described situation, and an object of the present invention is to provide a monitoring target person monitoring device, a monitoring target person monitoring method, and a monitoring target person monitoring system configured so that reduction in occurrence of a theft etc. and recording of a state in nursing care or assistance can be realized while the privacy of a monitoring target person is taken into consideration.

The monitoring target person monitoring device, the monitoring target person monitoring method, and the monitoring target person monitoring system according to the present invention sense a predetermined event regarding a monitoring target person to notify the event; acquire an image including at least a video; determine, based on the acquired image, whether or not multiple persons are on the image; and start, in a case where it is determined that the multiple persons are on the image, storing the acquired video to store the video in a video storage.

The above-described object, features, and advantageous effects of the present invention and other objects, features, and advantageous effects of the present invention will be apparent from detailed description below and attached drawings.

### Brief Description of Drawings

Fig. 1 is a diagram of a configuration of a monitoring target person monitoring system in an embodiment.
Fig. 2 is a diagram of a configuration of a sensor device in the monitoring target person monitoring system illustrated in Fig. 1.
Fig. 3 is a diagram of a configuration of a management server device in the monitoring target person monitoring system illustrated in Fig. 1.
Fig. 4 is a view of a configuration of a server-side monitoring information table stored in the management server device illustrated in Fig. 3.
Fig. 5 is a view of a configuration of an inter-device information table stored in the management server device illustrated in Fig. 3.
Fig. 6 is a view of a configuration of a server-side sensor information table stored in the management server device illustrated in Fig. 3.
Fig. 7 is a diagram of a configuration of a mobile terminal device in the monitoring target person monitoring system illustrated in Fig. 1.
Fig. 8 is a flowchart of operation of the sensor device illustrated in Fig. 2.
Fig. 9 is a flowchart of operation of the server device illustrated in Fig. 3.
Fig. 10 is a flowchart of operation of the mobile terminal device illustrated in Fig. 7.
Fig. 11 is a view of one example of a standby screen displayed on the mobile terminal device illustrated in Fig. 7.
Fig. 12 is a view of one example of a monitoring information screen displayed on the mobile terminal device illustrated in Fig. 7.
Fig. 13 is a view of one example of a nurse call receiving screen displayed on the mobile terminal device illustrated in Fig. 7.
Fig. 14 is a view of one example of a submenu screen displayed on the mobile terminal device illustrated in Fig. 7.
Fig. 15 is a view of one example of a video selection screen displayed on the mobile terminal device illustrated in Fig. 7.
Fig. 16 is a view of one example of a video display screen displayed on the mobile terminal device illustrated in Fig. 7.

### Description of Embodiments

Hereinafter, one embodiment of the present invention will be described with reference to the drawings. Note that the same reference numerals are used to represent the same configurations in each figure, and as necessary, description thereof will be omitted. In the present specification, a reference numeral without an index is used as a collective term, and a reference numeral with an index is used to indicate an individual configuration.

A monitoring target person monitoring system in the embodiment is for monitoring a monitoring target person (a tracking target person) Ob as a monitoring target (a tracking target) to be monitored (to be tracked), and includes a terminal device and a monitoring target person monitoring device communicably connected to the terminal device. The monitoring target person monitoring device in the monitoring target person monitoring system senses a predetermined event regarding the monitoring target person as the monitoring target to notify the event to the terminal device. The monitoring target person monitoring device includes an image acquisitor configured to acquire an image including at least a video, a video storage configured to store the video acquired by the image acquisitor, a multiple-person determiner configured to determine, based on the image acquired by the image acquisitor, whether or not multiple persons are on the image, and a video storage processor configured to start, in a case where the multiple-person determiner determines that the multiple persons are on the image, storing the video acquired by the image acquisitor to store the video in the video storage. A single device may integrally form this monitoring target person monitoring device. However, in the present embodiment, the monitoring target person monitoring device includes a sensor device and a management server device communicably connected to each of the sensor device and the terminal device. Thus, two types of devices separately form the monitoring target person monitoring device. In this aspect, the video storage processor preferably includes, for example, a first video processor configured to start, in a case where the multiple-person determiner determines that the multiple persons are on the image, delivering the video acquired by the image acquisitor, and a second video processor configured to store the video delivered by the first video processor in the video storage. The sensor device includes the image acquisitor, the multiple-person determiner, and the first video processor. The first video processor delivers the video to the management server device, and the management server device includes the video storage and the second video processor. In such a monitoring target person monitoring device, the video storage processor preferably terminates storage of the video after a lapse of a predetermined time after the start of storage of the video. In the above-described case, the first video processor preferably terminates delivery of the video after a lapse of a predetermined time after the start of delivery of the video. Note that the terminal device may be a single type of device. However, in the present embodiment, the terminal device includes two types of devices including a fixed terminal device and a mobile terminal device. A main difference between the fixed terminal device and the mobile terminal device is that the fixed terminal device operates in a fixed manner, whereas the mobile terminal device operates with the mobile terminal device being carried by a monitoring person (a user) such as a nurse or a caregiver. The fixed terminal device and the mobile terminal device are substantially similar to each other, and therefore, the mobile terminal device will be mainly described in the embodiment below.

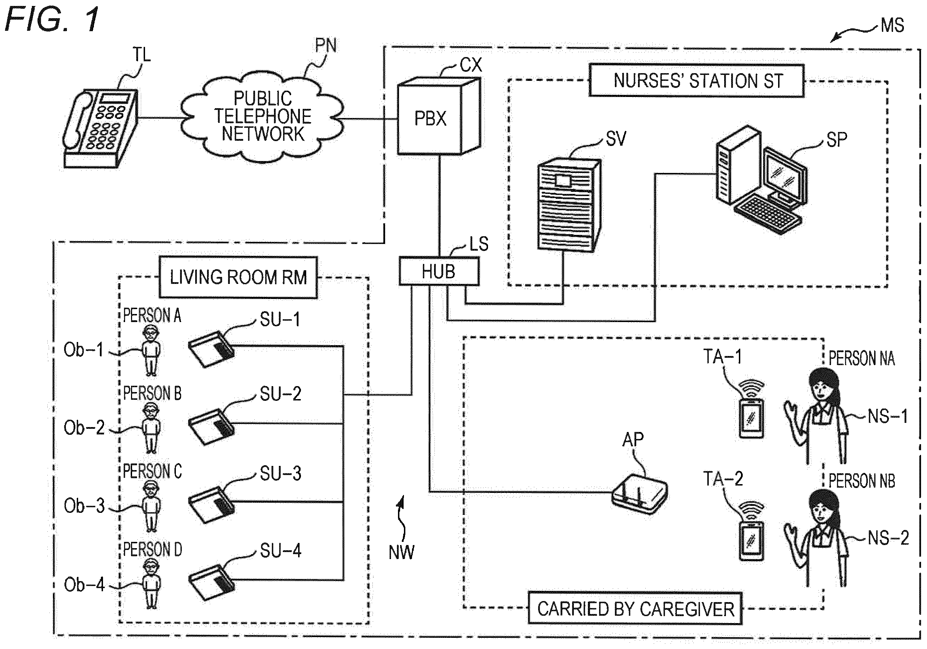

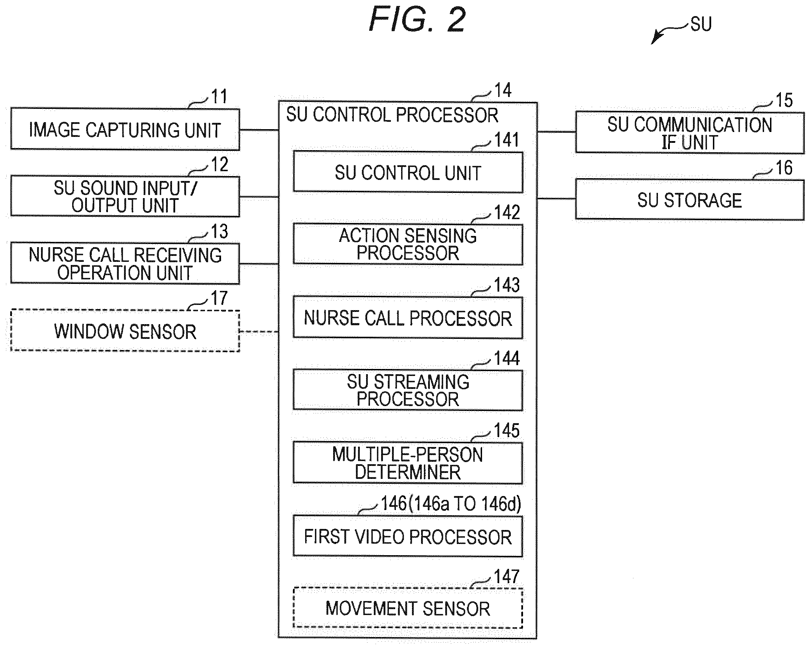

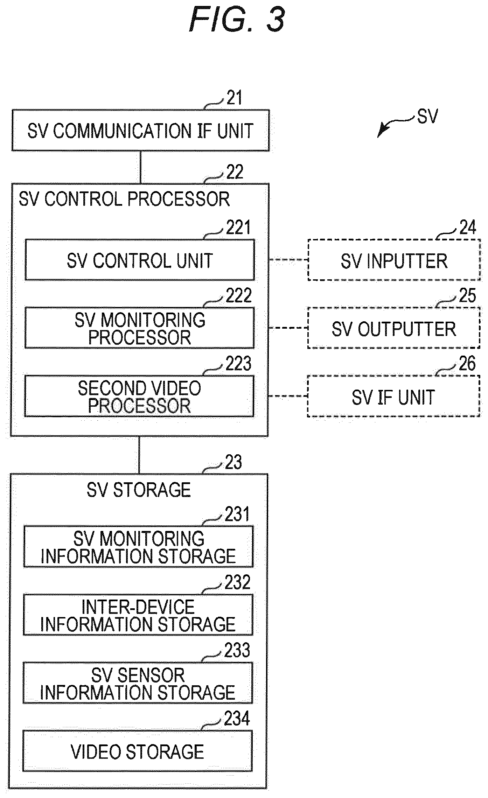

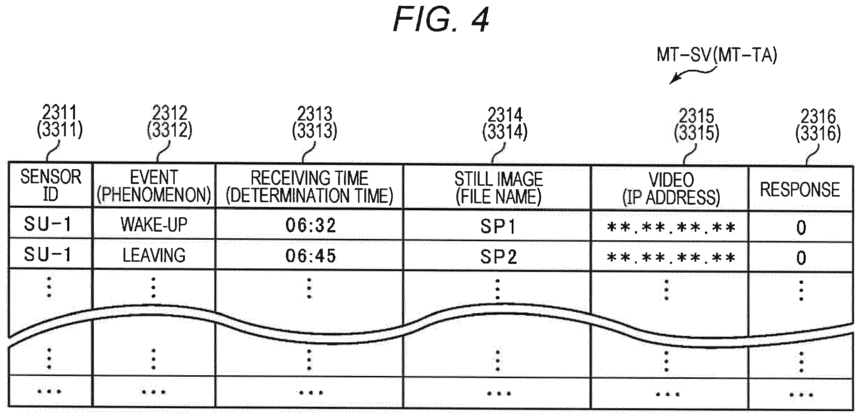

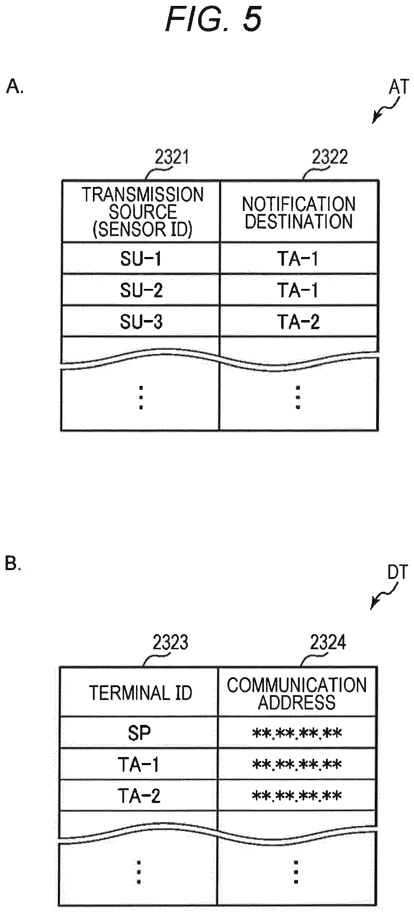

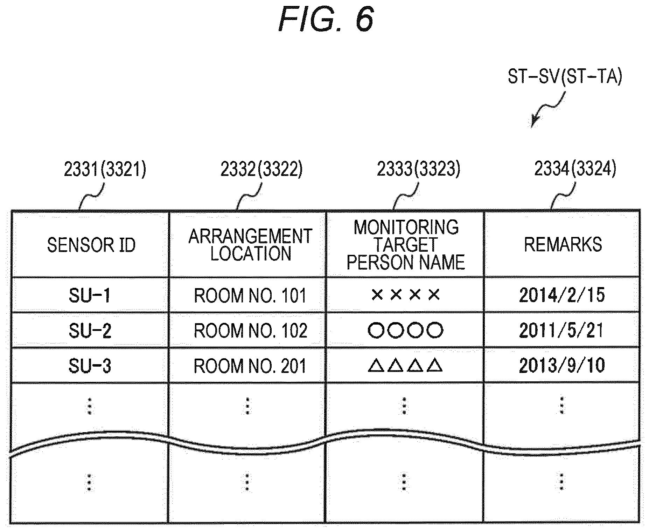

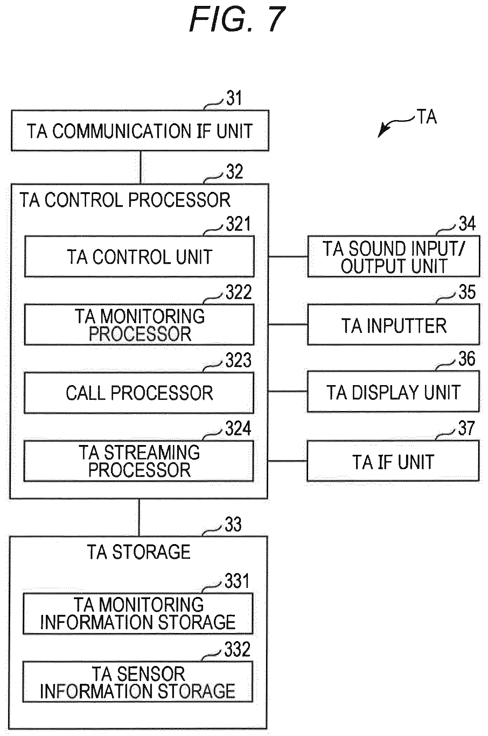

Fig. 1 is a diagram of a configuration of the monitoring target person monitoring system in the embodiment. Fig. 2 is a diagram of a configuration of a sensor device in the monitoring target person monitoring system of the embodiment. Fig. 3 is a diagram of a configuration of a management server device in the monitoring target person monitoring system of the embodiment. Fig. 4 is a view of a configuration of a server-side monitoring information table stored in the management server device illustrated in Fig. 3. Fig. 5 is a view of a configuration of an inter-device information table stored in the management server device illustrated in Fig. 3. Fig. 5A illustrates a configuration of a notification destination correspondence information table of the inter-device information table, and Fig. 5B illustrates a configuration of a communication address correspondence information table of the inter-device information table. Fig. 6 is a view of a configuration of a server-side sensor information table stored in the management server device illustrated in Fig. 3. Fig. 7 is a diagram of a configuration of a mobile terminal device in the monitoring target person monitoring system of the embodiment.

More specifically, the monitoring target person monitoring system MS in the embodiment includes, as illustrated in Fig. 1, one or more sensor devices SU (SU-1 to SU-4), the management server device SV, a fixed terminal device SP, one or more mobile terminal devices TA (TA-1, TA-2), and a private branch exchange (PBX) CX, for example. These components are, with or without a wire, communicably connected together via a net (a network, a communication line) NW such as a local area network (LAN). The network NW may include a relay configured to relay a communication signal, such as a repeater, a bridge, or a router. In an example illustrated in Fig. 1, the multiple sensor devices SU-1 to SU-4, the management server device SV, the fixed terminal device SP, the multiple mobile terminal devices TA-1, TA-2, and the private branch exchange CX are communicably connected together via the LAN (e.g., a LAN according to IEEE 802.11 standards) NW with a combination of wired and wireless connections including a line concentrator (a hub HUB) LS of a L2 switch and an access point AP. More specifically, the multiple sensor devices SU-1 to SU-4, the management server device SV, the fixed terminal device SP, and the private branch exchange CX are connected to the line concentrator LS, and the multiple mobile terminal devices TA-1, TA-2 are connected to the line concentrator LS via the access point AP. Using an Internet protocol group of, e.g., a transmission control protocol (TCP) and an Internet protocol (IP), the network NW forms a so-called intranet.

The monitoring target person monitoring system MS is arranged at an optional location according to a monitoring target person Ob. The monitoring target person (a tracking target person) Ob is, for example, a person needing nursing care due to a disease, an injury, etc., a person needing elderly care due to, e.g., a decrease in physical performance, or an unmarried person living on one's own. Specifically, considering availability of early recognition and early response, the monitoring target person Ob is preferably a person needing recognition of a predetermined disadvantageous event such as an abnormal state in a case where such an event occurs in the person. Thus, the monitoring target person monitoring system MS is, according to the type of monitoring target person Ob, suitably arranged at a building such as a hospital, a welfare facility for the elderly, or a residence. In the example illustrated in Fig. 1, the monitoring target person monitoring system MS is arranged at a building of an elderly care facility with multiple rooms including, for example, multiple living spaces RM where multiple monitoring target persons Ob settle and a nurses' station.

The sensor device SU is a device having, for example, the communication function of communicating with other devices SV, SP, TA via the network NW and configured to notify the management server device SV of a predetermined event (phenomenon) regarding the monitoring target person Ob. The predetermined event (phenomenon) is preferably a predetermined event needing to be handled. In the present embodiment, the predetermined event is, for example, predetermined action set in advance for the monitoring target person Ob and a nurse call. Thus, in the present embodiment, the sensor devices SU is a device configured to sense the predetermined action of the monitoring target person Ob to notify (transmit), as one example of the predetermined event, a sensing result to the management server device SV, to receive the nurse call to notify (transmit), as another example of the predetermined event, the nurse call to the management server device SV, to perform voice call with the terminal device SP, TA, and to generate an image including a video to deliver the video to the terminal device SP, TA. In the present embodiment, the sensor device SU determines whether or not multiple persons are on an image of an image capturing target. In a case where it is, as a result of determination, determined that the multiple persons are on the image of the image capturing target, video delivery begins to deliver the video for a predetermined time for the purpose of recording (storing) the video. In a case where it is not determined that the multiple persons are on the image of the image capturing target, no video is delivered. Such a sensor device SU includes, as illustrated in Fig. 2, an image capturing unit 11, a sensor-side sound input/output unit (a SU sound input/output unit) 12, a nurse call receiving operation unit 13, a sensor-side control processor (a SU control processor) 14, a sensor-side communication interface unit (a SU communication IF unit) 15, and a sensor-side storage (a SU storage) 16, for example.

The image capturing unit 11 is a device connected to the SU control processor 14 and configured to generate an image (image data) according to the control of the SU control processor 14. The image includes a still image (still image data) and a video (video data). The image capturing unit 11 is arranged so that a space (a residential space, the living space (room) RM as an arrangement location in the example illustrated in Fig. 1) where residency of the monitoring target person Ob as a monitoring target to be monitored is expected can be monitored. The image capturing unit 11 captures an image of the residential space as an image capturing target from above, and generates an image (image data) of the image capturing target from a bird's-eye view. Then, the image capturing unit 11 outputs the image (a target image) of the image capturing target to the SU control processor 14. There is a high probability that an image of the entirety of the monitoring target person Ob targeted for monitoring can be captured, and therefore, the image capturing unit 11 is preferably arranged so that the image of the image capturing target can be captured from right above a preset expected head position (normally, a pillow arrangement position) at which the head of the monitoring target person Ob is expected to be positioned on bedding (e.g., a bed) on which the monitoring target person Ob lies. The sensor device SU acquires, using the image capturing unit 11, the image of the monitoring target person Ob captured from above, and preferably the image captured from right above the expected head position.

The image capturing unit 11 described above may be a device configured to generate a visible light image, but in the present embodiment, is a device configured to generate an infrared light image so that the monitoring target person Ob can be monitored relatively in the dark. For example, in the present embodiment, the image capturing unit 11 is a digital infrared camera including, for example, an imaging optical system configured to form an infrared optical image of the image capturing target on a predetermined imaging surface, an area image sensor arranged such that a light receiving surface thereof is coincident with the imaging surface and configured to convert the infrared optical image of the image capturing target into an electric signal, and an image processor configured to perform image processing for the output of the area image sensor to generate image data as data indicating the infrared image of the image capturing target. In the present embodiment, the imaging optical system of the image capturing unit 11 is preferably a wide-angle optical system (a so-called wide-angle lens (including a fish-eye lens)) having such an angle of view that the image of the entirety of the living space RM where such a system is arranged can be captured.

The SU sound input/output unit 12 is a circuit configured to input/output sound. That is, the SU sound input/output unit 12 is a circuit connected to the SU control processor 14 and configured to generate and output, according to the control of the SU control processor 14, sound corresponding to an electric signal indicating sound, and is a circuit configured to acquire external sound to input the sound to the sensor device SU. The SU sound input/output unit 12 includes, for example, a speaker configured to convert a sound electric signal (sound data) into a sound machine vibration signal (an acoustic signal), and a microphone configured to convert a sound machine vibration signal in an audible range into an electric signal. The SU sound input/output unit 12 outputs an electric signal indicating the external sound to the SU control processor 14, and converts an electric signal input from the SU control processor 14 into a sound machine vibration signal to output the resultant signal.

The nurse call receiving operation unit 13 is a switch circuit connected to the SU control processor 14 and configured to input the nurse call to the sensor device SU, such as a push-button switch. Note that the nurse call receiving operation unit 13 may be connected to the SU control processor 14 with a wire, or may be connected to the SU control processor 14 via near field communication according to, e.g., Bluetooth (registered trademark) standards.

The SU communication IF unit 15 is a communication circuit connected to the SU control processor 14 and configured to perform communication according to the control of the SU control processor 14. The SU communication IF unit 15 generates, according to the communication protocol used in the network NW of the monitoring target person monitoring system MS, a communication signal containing transfer target data input from the SU control processor 14, thereby transmitting the generated communication signal to other devices SV, SP, TA via the network NW. The SU communication IF unit 15 receives a communication signal from other devices SV, SP, TA via the network NW to extract data from the received communication signal, and converts the extracted data into data in a form processable by the SU control processor 14 to output the resultant data to the SU control processor 14. The SU communication IF unit 15 includes, for example, a communication interface circuit according to the IEEE 802.11 standards.

The SU storage 16 is a circuit connected to the SU control processor 14 and configured to store, according to the control of the SU control processor 14, various predetermined programs and various types of predetermined data. Various predetermined programs described above include control processing programs such as a SU control program for controlling each unit of the sensor device SU according to the function thereof and a SU monitoring processing program for executing predetermined information processing regarding monitoring of the monitoring target person Ob. The SU monitoring processing program includes, for example, an action sensing processing program for sensing predetermined action of the monitoring target person Ob to notify, as one example of the predetermined event (phenomenon), a sensing result to the predetermined terminal device SP, TA via the management server device SV, a nurse call processing program for notifying, as another example of the predetermined event, receiving of the nurse call by the nurse call receiving operation unit 13 to the management server device SV to perform voice call with the terminal device SP, TA by means of, e.g., the SU sound input/output unit 12, a SU streaming processing program for delivering, by streaming, the video generated in the image capturing unit 11 to the terminal device SP, TA having requested such a video, a multiple-person determination program for determining whether or not the multiple persons are on the image based on the image acquired by the image capturing unit 11, and a first video processing program for starting delivery of the video acquired by the image capturing unit 11 in a case where the multiple-person determination program determines that the multiple persons are on the image. Various types of predetermined data described above include data necessary for execution of each program, such as a sensor device identifier (a sensor ID) of a subject unit as an identifier for specifying and identifying the sensor device SU and a communication address of the management server device SV. The SU storage 16 includes, for example, a read only memory (ROM) as a non-volatile storage element and an electrically erasable programmable read only memory (EEPROM) as a rewritable non-volatile storage element. Moreover, the SU storage 16 includes, for example, a random access memory (RAM) as a so-called working memory of the SU control processor 14 for storing data etc. generated during execution of the predetermined program.

The SU control processor 14 is a circuit configured to control each unit of the sensor device SU according to the function thereof, thereby notifying the predetermined event (phenomenon), which needs to be handled, regarding the monitoring target person Ob to the management server device SV. More specifically, in the present embodiment, the SU control processor 14 senses the predetermined action of the monitoring target person Ob to notify (inform, transmit), as one example of the predetermined event, such a sensing result to the management server device SV; receives the nurse call to notify (inform, transmit), as another example of the predetermined event, the nurse call to the management server device SV; performs voice call with the terminal device SP, TA; generates the image containing the video to deliver the video to the terminal device SP, TA; and for determining an independency, temporally continuously transmits the image to the management server device. In the present embodiment, the SU control processor 14 determines whether or not the multiple persons are on the image of the image capturing target. In a case where it is, as a result of determination, determined that the multiple persons are on the image of the image capturing target, delivery of the video begins to deliver the video to the management server device SV for the predetermined time for the purpose of recording (storing) the video. In a case where it is not determined that the multiple persons are on the image of the image capturing target, no video is delivered. The SU control processor 14 includes, for example, a central processor (CPU) and peripheral circuits thereof. By execution of the control processing programs, the SU control processor 14 functionally includes a sensor-side control unit (a SU control unit) 141, an action sensing processor 142, a nurse call processor 143, a sensor-side streaming processor (a SU streaming processor) 144, a multiple-person determiner 145, and a first image processor 146 (146a to 146e).

The SU control unit 141 is configured to control each unit of the sensor device SU according to the function thereof and govern control of the entirety of the sensor device SU.

The action sensing processor 142 is configured to sense the preset predetermined action of the monitoring target person Ob to notify, as one example of the predetermined event, such a sensing result to the management server device SV. More specifically, in the present embodiment, the predetermined action includes, for example, four types of action including sitting-up of the monitoring target person Ob, leaving of the monitoring target person Ob from the bedding, falling of the monitoring target person Ob from the bedding, and overturning of the monitoring target person Ob. For example, the action sensing processor 142 detects the head of the monitoring target person Ob based on the target image captured by the image capturing unit 11, and senses sitting-up, leaving, falling, and overturning of the monitoring target person Ob based on a temporal change in the size of the detected head of the monitoring target person Ob. More specifically, a location region of the bedding BD and first to third thresholds Th1 to Th3 are, as ones of various types of predetermined data, first stored in advance in the SU storage 16. The first threshold Th1 is a value for identifying the size of the head in a lying posture and the size of the head in a seating posture in the location region of the bedding BD. The second threshold Th2 is a value for identifying whether or not the size is the size of the head in a standing posture in the living space RM excluding the location region of the bedding BD. The third threshold Th3 is a value for identifying whether or not the size is the size of the head in the lying posture in the living space RM excluding the location region of the bedding BD. The action sensing processor 142 extracts a moving body region as a person region of the monitoring target person Ob from the target image by, e.g., a background differencing technique or a frame differencing technique. Next, the action sensing processor 142 extracts a head region of the monitoring target person Ob from the extracted moving body region by circular or ellipse Hough transform, pattern matching using a head model prepared in advance, or a neural network having learnt for head detection, for example. Then, the action sensing processor 142 senses sitting-up, leaving, falling, and overturning from the extracted position and size of the head. For example, in a case where the extracted position of the head is within the location region of the bedding BD and the extracted size of the head temporally changes from the size in the lying posture to the size in the seating posture by means of the first threshold Th1, the action sensing processor determines as sitting-up, and senses sitting-up. For example, in a case where the extracted position of the head temporally changes from that within the location region of the bedding BD to that outside the location region of the bedding and the extracted size of the head temporally changes from a certain size to the size in the standing posture by means of the second threshold Th2, the action sensing processor 142 determines as leaving, and senses leaving. For example, in a case where the extracted position of the head temporally changes from that within the location region of the bedding BD to that outside the location region of the bedding BD and the extracted size of the head temporally changes from a certain size to the size in the lying posture by means of the third threshold Th3, the action sensing processor 142 determines as falling, and senses falling. For example, in a case where the extracted position of the head is within the living space RM excluding the location region of the bedding BD and the extracted size of the head temporally changes from a certain size to the size in the lying posture by means of the third threshold Th3, the action sensing processor 142 determines as overturning, and senses overturning.

When sensing the predetermined action as described above, the action sensing processor 142 notifies a first event notification communication signal for notifying the event to the management server device SV via the SU communication IF unit 15, the first event notification communication signal containing event information (phenomenon information) on the contents of the predetermined event (phenomenon) regarding the monitoring target person Ob. More specifically, the action sensing processor 142 transmits the communication signal (the first event notification communication signal) to the management server device SV via the SU communication IF unit 15, the communication signal containing the sensor ID of the subject unit, the event information on the contents of the event, and the target image used for sensing the predetermined action. In the present embodiment, the event information is one or more of sitting-up, leaving, falling, overturning, and the nurse call (NC). In this embodiment, the action sensing processor 142 allows the first event notification communication signal to contain, as the event information, one or more of sensed sitting-up, leaving, falling, and overturning. The image may be at least one of the still image or the video. In the present embodiment, the still image is first informed, and then, the video is delivered according to a user's request, as will be described later. Note that the video may be first delivered. Alternatively, the still image and the video may be transmitted, and then, may be displayed on divided screens of the terminal device SP, TA.

The nurse call processor 143 is configured to notify, in the case of receiving the nurse call by the nurse call receiving operation unit 13, the management server device SV of the first event notification communication signal containing such receiving as another example of the predetermined event and to perform voice call with the terminal device SP, TA by means of the SU sound input/output unit 12 etc. More specifically, upon input operation for the nurse call receiving operation unit 13, the nurse call processor 143 transmits the first event notification communication signal to the management server device SV via the SU communication IF unit 15, the first event notification communication signal containing the sensor ID of the subject unit and the nurse call as the event information. The nurse call processor 143 uses the SU sound input/output unit 12 etc. to perform voice call with the terminal device SP, TA according to, e.g., a voice over Internet protocol (VoIP).

The SU streaming processor 144 is configured to deliver, in a case where a video delivery request has been received from the fixed terminal device SP or the mobile terminal device TA via a communication IF unit 3, the video (e.g., a live video) generated by the image capturing unit 11 to the fixed terminal device SP or the mobile terminal device TA having made the request via the SU communication IF unit 15 by streaming reproduction.

The multiple-person determiner 145 is configured to determine, based on the image acquired by the image capturing unit 11, whether or not the multiple persons are on the image. More specifically, the multiple-person determiner 145 extracts the moving body region as the person region from the target image acquired by the image capturing unit 11 by, e.g., the background differencing technique or the frame differencing technique to determine whether or not there are multiple extracted moving body regions, thereby determining whether or not the multiple persons are on the image. In a case where it is, as a result of determination, determined that there are multiple extracted moving body regions (two or more regions), the multiple-person determiner 145 determines that the multiple persons are on the target image. In a case where there is one or less extracted moving body region, the multiple-person determiner 145 determines that no multiple persons are on the target image. Note that the person region is assumed as being equal to or larger than a predetermined first size (a first size, a first area) and equal to or smaller than a predetermined second size (a second size, a second area). Thus, the first and second sizes may be determined in advance from multiple samples, and the multiple-person determiner 145 may take, from the extracted moving body regions, a moving body region equal to or larger than the first size and equal to or smaller than the second size as the person region. That is, the multiple-person determiner 145 ignores a moving body region, which is smaller than the first size, of the extracted moving body regions, and does not count such a moving body region as the person region. Moreover, the multiple-person determiner 145 ignores a moving body region, which exceeds the second size, of the extracted moving body regions, and does not count such a moving body region as the person region.

The first video processor 146 is configured to start delivery of the video acquired by the image capturing unit 11 to the management server device SV in a case where the multiple-person determiner 145 determines that the multiple persons are on the target image. More specifically, the first video processor 146 sequentially generates, for a preset predetermined time, a communication signal (a video delivery communication signal) containing the sensor ID of the subject unit and a predetermined number of frames of the video acquired by the image capturing unit 11, and transmits each of the sequentially-generated video delivery communication signals to the management server device SV. Of these sequentially-generated video delivery communication signals, the last video delivery communication signal contains information (delivery end information) indicating last delivery. As will be described later, when receiving each video delivery communication signal, the management server device SV stores (records) the contained video of such a signal in association with the sensor ID. Note that each video delivery communication signal may contain an instruction (a command) for storing (recording) the video in the management server device SV.

Fig. 1 illustrates, as one example, four first to fourth sensor devices SU-1 to SU-4. The first sensor device SU-1 is arranged in a living space RM-1 (not shown) of a person A Ob-1 as one of the monitoring target persons Ob, the second sensor device SU-2 is arranged in a living space RM-2 (not shown) of a person B Ob-2 as one of the monitoring target persons Ob, the third sensor device SU-3 is arranged in a living space RM-3 (not shown) of a person C Ob-3 as one of the monitoring target persons Ob, and the fourth sensor device SU-4 is arranged in a living space RM-4 (not shown) of a person D Ob-4 as one of the monitoring target persons Ob.

The management server device SV has the communication function of communicating with other devices SU, TA, SP via the network NW. The management server device SV is a device configured to manage, when receiving notification of the predetermined event from the sensor device SU, information (monitoring information (in the present embodiment, e.g., the predetermined event (the type of predetermined action sensed by the sensor device SU or the nurse call received by the sensor device SU), the image (the still image and the video) of the monitoring target person Ob, and the time of receiving the notification)) regarding monitoring of the monitoring target person Ob, to notify (re-notify, re-inform, transmit) the predetermined event to the predetermined terminal device SP, TA, to provide a client (in the present embodiment, the terminal device SP, TA, etc.) with data corresponding to a request from the client, and to manage the entirety of the monitoring target person monitoring system MS. In the present embodiment, when receiving the video delivery communication signal from the sensor device SU via the network NW, the management server device SV starts storing (recording) the video contained in the received video delivery communication signal, and stores (records) the video in association with the sensor device SU (the sensor ID, and in other words, the monitoring target person Ob in the living space RM where the sensor device SU is arranged). As illustrated in, e.g., Fig. 3, the management server device SV includes a server-side communication interface unit (a SV communication IF unit) 21, a server-side control processor (a SV control processor) 22, and a server-side storage (a SV storage) 23.

As in the SU communication IF unit 15, the SV communication IF unit 21 is a communication circuit connected to the SV control processor 22 and configured to perform communication according to the control of the SV control processor 22. The SV communication IF unit 21 includes, for example, a communication interface circuit according to the IEEE 802.11 standards.

The SV storage 23 is a circuit connected to the SV control processor 22 and configured to store various predetermined programs and various types of predetermined data according to the control of the SV control processor 22. Various predetermined programs described above include control processing programs such as a SV control program for controlling each unit of the management server device SV according to the function thereof, a SV monitoring processing program for executing predetermined information processing regarding monitoring of the monitoring target person Ob, and a second video processing program for storing, in a later-described video storage 234, the video delivered by the first video processor 146 of the sensor device SU via the network NW. Various types of predetermined data described above include data necessary for execution of each program, such as a server identifier (a server ID) of a subject unit for specifying the management server device SV and identifying the management server device SV, the monitoring information on the monitoring target person Ob, inter-device information indicating information among the devices SU, SP, TA such as predetermined phenomenon notification destinations, the sensor information on the sensor device SU, and the video delivered from the sensor device SU. For storing each of the monitoring information, the inter-device information, the sensor information, and the video, the SV storage 23 functionally includes a server-side monitoring information storage (a SV monitoring information storage) 231, an inter-device information storage 232, a server-side sensor information storage (a SV sensor information storage) 234, and the video storage 234.

The SV monitoring information storage 231 is configured to store the monitoring information on the monitoring target person Ob, the monitoring information being received from or transmitted to each device SU, SP, TA. More specifically, in the present embodiment, the sensor ID, the event information (the event information including, in the present embodiment, sitting-up, leaving, falling, overturning, and the nurse call), the receiving time, the target image (the still image and the video), and the presence or absence of a response are, in the SV monitoring information storage 231, stored as the monitoring information in association with each other based on each type of information contained in the communication signal such as the first event notification communication signal and a later-described response receiving notification communication signal.

In the present embodiment, the monitoring information is stored in a table format in a SV monitoring information storage 321. As illustrated in Fig. 4, a server-side monitoring information table (a SV monitoring information table) MT-SV registering the monitoring information includes, for example, a sensor ID field 2311 for registering the sensor ID contained in the communication signal received from each device SU, SP, TA, an event field (an event field) 2312 for registering the event information contained in the received communication signal, a receiving time field 2313 for registering the receiving time of the received communication signal, a still image field 2314 for registering the still image contained in the received communication signal, a video field 2315 for registering a communication address (e.g., an IP address) of the sensor device SU corresponding to the sensor ID contained in the received communication signal, and a response field 2316 for registering the presence or absence of receiving of the response to the event information contained in the received communication signal. The SV monitoring information table MT-SV includes a record for each received communication signal (each event). In the still image field 2314, the image data of the still image may be recorded, or a file name of the image data of the still image may be registered, for example. As will be described later, a flag (a response flag) indicating whether or not an indication ("RESPOND") of intention to handle (respond, treat, take measures) the event information contained in the received communication signal is received by the terminal device SP, TA is registered in the response field 2316. For example, in the present embodiment, a response flag of "1" meaning that the indication ("RESPOND") of intention to handle the event information (the event information registered in the event field 2312) contained in the received communication signal has been received by the terminal device SP, TA or a response flag of "0" meaning that the indication ("RESPOND") of intention to handle the event information contained in the received communication signal is not received by the terminal device SP, TA is registered in the response field 2316. Note that a response flag of "0" meaning unreceiving is registered as a default in the response field 2316. Note that in a case where the first event notification communication signal contains the sensing time of sensing the predetermined action or the nurse call receiving time of receiving the nurse call, the sensing time or the nurse call receiving time may be registered instead of the receiving time.

In the present embodiment, the inter-device information storage 232 stores, as the inter-device information, the correspondence (the notification destination correspondence), which indicates a notification destination (a re-notification destination, a re-informing destination, a transmission destination) of, e.g., the first event notification communication signal transmitted from the sensor device SU, between the sensor ID as a transmission source and a terminal ID as an informing destination (the re-informing destination) and a correspondence (a communication address correspondence) between the ID (the sensor ID, the terminal ID) of each device SU, SP, TA and the communication address thereof, for example. The terminal ID is a terminal identifier for specifying the terminal device SP, TA and identifying the terminal device SP, TA.

In the present embodiment, each of the notification destination correspondence and the communication address correspondence is stored in a table format in the inter-device information storage 232. As illustrated in, e.g., Fig. 5A, a notification destination correspondence information table AT for registering the notification destination correspondence includes a transmission source field 2321 for registering the sensor ID of the sensor device SU as the transmission source, and a notification destination field 2322 for registering the terminal ID of the terminal device SP, TA as the transmission destination to which the communication signal notified from the sensor device SU corresponding to the sensor ID registered in the transmission source field 2321 is transmitted. The notification destination correspondence information table AT includes a record for each sensor ID (each sensor device SU). As illustrated in, e.g., Fig. 5B, a communication address correspondence information table DT for registering the communication address correspondence includes a terminal ID field 2323 for registering the terminal ID of the terminal device SP, TA, and a communication address field 2324 for registering the communication address of the terminal device SP, TA corresponding to the terminal ID registered in the terminal ID field 2323. The communication address correspondence information table DT includes a record for each terminal ID (each terminal device SP, TA).

Note that each of the sensor ID, the server ID, and the terminal ID may be, e.g., a serial number including a predetermined symbol string, or may be, e.g., a communication address (in this case, the communication address correspondence can be omitted).

The SV sensor information storage 233 is configured to store the sensor information. In the present embodiment, the sensor information is information regarding the sensor device SU, and is information with an association between the sensor ID of the sensor device SU and a monitoring target person name of the monitoring target person Ob.

In the present embodiment, such sensor information is stored in a table format in the SV sensor information storage 233. More specifically, a sensor information table ST-SV for registering the sensor information includes, as illustrated in, e.g., Fig. 6, a sensor ID field 2331 for registering the sensor ID, an arrangement location field 2332 for registering the arrangement location of the sensor device SU having the sensor ID registered in the sensor ID field 2331, a monitoring target person name field 2333 for registering the monitoring target person name of the monitoring target person Ob (i.e., the monitoring target person Ob at the arrangement location of the sensor device SU having the sensor ID registered in the sensor ID field 2331) monitored by the sensor device SU having the sensor ID registered in the sensor ID field 2331, and a remarks field 2334 for registering remarks regarding the sensor device SU having the sensor ID registered in the sensor ID field 2331, the arrangement location of the sensor device SU, and remarks regarding the monitoring target person Ob for the sensor device SU. The sensor information table ST-SV has a record for each sensor ID (i.e., each sensor device SU).

The video storage 234 is configured to store (record) the video delivered in the video delivery communication signal from the sensor device SU in association with the sensor ID (the sensor ID contained in the received video delivery communication signal) of the sensor device SU having delivered the video. More specifically, the video storage 234 stores the video with a file name, and stores the file name of the video in association with the sensor ID.

The SV control processor 22 is a circuit configured to control each unit of the management server device SV according to the function thereof, to manage the monitoring information regarding monitoring of the monitoring target person Ob when receiving notification of the predetermined phenomenon from the sensor device SU, to notify (re-notify, re-inform, transmit) the predetermined event to the predetermined terminal device SP, TA, to store the video through storage of the video delivered from the sensor device SU, to provide the client with the data corresponding to the request from the client, and to manage the entirety of the monitoring target person monitoring system MS. The SV control processor 22 includes, for example, a CPU and peripheral circuits thereof. By execution of the control processing programs, the SV control processor 22 functionally includes a server-side control unit (a SV control unit) 221, a server-side monitoring processor (a SV monitoring processor) 222, and a second video processor 223.

The SV control unit 221 is configured to control each unit of the management server device SV according to the function thereof and govern control of the entirety of the management server device SV.

The SV monitoring processor 222 is configured to manage the monitoring information regarding monitoring of the monitoring target person Ob when receiving notification of the predetermined event from the sensor device SU and to inform the predetermined event to the predetermined terminal device SP, TA. More specifically, when receiving the first event notification communication signal from the sensor device SU, the SV monitoring processor 222 stores (records) the monitoring information regarding monitoring of the monitoring target person Ob in the SV monitoring information storage 231, the monitoring information being contained in the received first event notification communication signal. The SV monitoring processor 222 selects (searches), from the notification destination correspondence stored in the inter-device information storage 232, a reporting destination (a re-reporting destination, a transfer destination, a transmission destination) corresponding to the sensor device SU having transmitted the received first event notification communication signal, and transmits a second event notification communication signal to the selected terminal device SP, TA. Such selection (searching processing) is performed based on the sensor ID corresponding to the sensor device SU having transmitted the received first event notification communication signal. In a case where the event information contained in the first event notification communication signal is the predetermined action (one or more of sitting-up, leaving, falling, and overturning), the second event notification communication signal contains the sensor ID, the event information, and the target image contained in the first event notification communication signal and the communication address as a video downloading destination corresponding to the sensor device SU having the sensor ID contained in the first event notification communication signal. The communication address is selected (searched) from the communication address correspondence based on the sensor ID corresponding to the sensor device SU having transmitted the received first event notification communication signal.

The second video processor 223 is configured to store, in the video storage 234, the video delivered by the first video processor 146 of the sensor device SU via the network NW in association with the sensor device SU. More specifically, when sequentially receiving a series of video delivery communication signals from the sensor device SU, the second video processor 223 brings the videos contained in the received series of video delivery communication signals into a single video file. The generated video file is stored with a file name in the video storage 234. The file name of the stored video file is, in the video storage 234, stored in association with the sensor ID contained in the received series of video delivery communication signals.

Note that if needed, the management server device SV may further include, as indicated by dashed lines in Fig. 3, a server-side inputter (a SV inputter) 24 connected to the SV control processor 22 and configured to input various commands, various types of data, etc., a server-side outputter (a SV outputter) 25 configured to output various commands or various types of data input from the SV inputter 24, the monitoring information regarding monitoring of the monitoring target person Ob, etc., and a server-side interface unit (a SV IF unit) 26 configured to perform data input to or data output from external equipment, for example.

The management server device SV described above may include, for example, a computer with a communication function.

The fixed terminal device SP has, for example, the communication function of communicating with other devices SU, SV, TA via the network NW, the display function of displaying predetermined information, and the input function of inputting a predetermined instruction or data. The fixed terminal device SP is equipment functioning as a user interface (UI) of the monitoring target person monitoring system MS by inputting the predetermined instruction or data to be provided to the management server device SV or the mobile terminal device TA or displaying the monitoring information obtained by the sensor device SU, for example. In the present embodiment, when the user (a monitoring person) specifies the sensor device SU (the sensor ID or the monitoring target person name) or the fixed terminal device SP receives a video request, the fixed terminal device SP requests the video captured by the specified sensor device SU from the management server device SV, and displays the video received from the management server device SV. The fixed terminal device SP described above may include, for example, a computer with a communication function. Note that the fixed terminal device SP as one example of the terminal device is operable as in the mobile terminal device TA, and in the present specification, the mobile terminal device TA as another example of the terminal device will be described.

The mobile terminal device TA has, for example, the communication function of communicating with other devices SV, SP, SU via the network NW, the display function of displaying predetermined information, the input function of inputting a predetermined instruction or data, and the calling function of performing voice call. The mobile terminal device TA is equipment for inputting the predetermined instruction or data to be provided to the management server device SV or the sensor devices SU, displaying the monitoring information obtained by the sensor device SU in response to notification from the management server device SV, or performing a response to the nurse call or speaking by voice call with the sensor device SU, for example. In the present embodiment, when the user (the monitoring person) specifies the sensor device SU (the sensor ID or the monitoring target person name) and the mobile terminal device TA receives a video request, the mobile terminal device TA requests, as in the fixed terminal device SP, the video captured by the specified sensor device SU from the management server device SV, and displays the video received from the management server device SV. In the present embodiment, the mobile terminal device TA described above includes, as illustrated in, e.g., Fig. 7, a terminal-side communication interface unit (a TA communication IF unit) 31, a terminal-side control processor (a TA control processor) 32, a terminal-side storage (a TA storage) 33, a terminal-side sound input/output unit (a TA sound input/output unit) 34, a terminal-side inputter (a TA inputter) 35, a terminal-side display unit (a TA display unit) 36, and a terminal-side interface unit (a TA IF unit) 37.

As in the SU sound input/output unit 12, the TA sound input/output unit 34 is a circuit connected to the TA control processor 32 and configured to acquire external sound to input such sound to the mobile terminal device TA, and is a circuit configured to generate and output, according to the control of the TA control processor 32, sound corresponding to an electric signal indicating sound.

The TA inputter 35 is a circuit connected to the TA control processor 32 and configured to receive predetermined operation to input such operation to the mobile terminal device TA, for example. The TA inputter 35 includes, for example, multiple input switches assigned to predetermined functions. The predetermined operation includes various types of operation necessary for monitoring, such as ID input operation for login, the operation of requesting and terminating voice call, the operation of requesting and terminating a live video, and the operation of inputting the indication ("RESPOND") of intention to respond (handle, reply), such as execution of rescue, nursing care, elderly care, and assistance, to the monitoring target person Ob in association with the notified event. The TA display unit 36 is a circuit connected to the TA control processor 32 and configured to display, according to the control of the TA control processor 32, predetermined operation contents input from the TA inputter 35 or the monitoring information (e.g., the predetermined event (the type of predetermined action sensed by the sensor device SU or the nurse call received by the sensor device SU), the image (the still image and the video) of the monitoring target person Ob, and the time of receiving the notification) regarding monitoring of the monitoring target person Ob monitored by the monitoring target person monitoring system MS. The TA display unit 36 is, for example, a display device such as a liquid crystal display (LCD) or an organic EL display. In the present embodiment, the TA inputter 35 and the TA display unit 36 form a touch panel. In this case, the TA inputter 35 is a position input device configured to detect and input an operation position by, e.g., a resistive film technique or an electrostatic capacitance technique. In this touch panel, the position input device is provided on a surface of the TA display unit 36, and one or more candidates for input contents inputtable to the TA display unit 36 are displayed. For example, when the user (the monitoring person) such as a nurse or a caregiver touches a display position at which an intended input content is displayed, such a position is detected by the position input device, and the display content displayed at the detected position is, as a user operation input content, input to the mobile terminal device TA.

The TA IF unit 37 is a circuit connected to the TA control processor 32 and configured to perform data input to or data output from external equipment according to the control of the TA control processor 32. The TA IF unit 37 includes, for example, an interface circuit using the Bluetooth (registered trademark) standards, an interface circuit configured to perform infrared communication according to, e.g., IrDA standards, and an interface circuit using USB standards.

As in the SU communication IF unit 15, the TA communication IF unit 31 is a communication circuit connected to the TA control processor 32 and configured to perform communication according to the control of the TA control processor 32. The TA communication IF unit 31 includes, for example, a communication interface circuit according to the IEEE 802.11 standards.

The TA storage 33 is a circuit connected to the TA control processor 32 and configured to store various predetermined programs and various types of predetermined data according to the control of the TA control processor 32. Various predetermined programs described above include control processing programs such as a TA control program for controlling each unit of the mobile terminal device TA according to the function thereof, a TA monitoring processing program for executing predetermined information processing regarding monitoring of the monitoring target person Ob, a call processing program for performing voice call with the sensor device SU by means of the TA sound input/output unit 34 etc., and a TA streaming processing program for receiving a video delivered from the sensor device SU to display the delivered video on the TA display unit 36 by streaming reproduction. Various types of predetermined data described above include data necessary for execution of each program, such as a terminal ID of a subject unit, screen information displayed on the TA display unit 36, the monitoring information on the monitoring target person Ob, and the sensor information regarding the sensor device SU. The TA storage 33 includes, for example, a ROM and an EEPROM. The TA storage 33 includes, for example, a RAM as a so-called working memory of the TA control processor 32 for storing data etc. generated during execution of the predetermined program. For storing each of the monitoring information and the sensor information, the TA storage 33 functionally includes a terminal-side monitoring information storage (a TA monitoring information storage) 331 and a terminal-side sensor information storage (a TA sensor information storage) 332.

The TA monitoring information storage 331 is configured to store the monitoring information. In the present embodiment, the TA monitoring information storage 331 stores, as the monitoring information, the sensor ID, the event information (in the present embodiment, the event information including sitting-up, leaving, falling, overturning, and the nurse call), and the communication address of the sensor device SU as the image/video downloading destination in the second event notification communication signal received from the management server device SV, the time of receiving the second event notification communication signal, and the presence or absence of handling in association with each other. More specifically, as illustrated in Fig. 4, the TA monitoring information storage 331 stores the monitoring information in a terminal-side monitoring information table (a TA monitoring information table) MT-TA similar to the SV monitoring information table MT-SV.

The TA sensor information storage 332 is configured to store the sensor information. The TA sensor information storage 332 is configured to store the sensor ID, the arrangement location, the monitoring target person name, and the remarks in association with each other. More specifically, the TA sensor information storage 332 stores, as illustrated in Fig. 6, the sensor information in a terminal-side sensor information table (a TA sensor information table) ST-TA similar to the SV sensor information table ST-SV.

The TA control processor 32 is a circuit configured to control each unit of the mobile terminal device TA according to the function thereof, to receive and display the monitoring information on the monitoring target person Ob, and to perform the response to the nurse call or speaking. The TA control processor 32 includes, for example, a CPU and peripheral circuits thereof. By execution of the control processing programs, the TA control processor 32 functionally includes a terminal-side control unit (a TA control unit) 321, a terminal-side monitoring processor (a TA monitoring processor) 322, a call processor 323, and a terminal-side streaming processor (a TA streaming processor) 324.

The TA control unit 321 is configured to control each unit of the mobile terminal device TA according to the function thereof and govern control of the entirety of the mobile terminal device TA.

The TA monitoring processor 322 is configured to execute predetermined information processing regarding monitoring of the monitoring target person Ob. More specifically, when receiving the second event notification communication signal transmitted by the management server device SV due to the first event notification communication signal transmitted by the sensor device SU, the TA monitoring processor 322 stores (records) the monitoring information on the monitoring target person Ob in the TA monitoring information storage 331 based on each type of information (each type of data) contained in the received second event notification communication signal. The TA monitoring processor 322 displays, on the TA display unit 36, a screen corresponding to each type of information contained in the received second event notification communication signal. When receiving predetermined input operation from the TA inputter 35, the TA monitoring processor 322 executes predetermined processing corresponding to such input operation.

The call processor 323 is configured to perform voice call with the sensor device SU by means of, e.g., the TA sound input/output unit 34. More specifically, the call processor 323 uses the TA sound input/output unit 34 etc. to perform, by, e.g., VoIP, voice call with the sensor device SU as the transmission source having transmitted the first event notification communication signal as a cause for transmission of the second event notification communication signal to the management server device SV.

The TA streaming processor 324 is configured to receive a video delivered from the sensor device SU to display the delivered video on the TA display unit 36 by streaming reproduction.

The mobile terminal device TA described above may include, for example, a portable communication terminal device such as a so-called tablet computer, a smartphone, or a mobile phone.

In the monitoring target person monitoring system MS described above, a monitoring target person monitoring device includes two devices, i.e., the sensor device SU and the management server device SV. Thus, the image capturing unit 11 of the sensor device SU corresponds to one example of an image acquisitor configured to acquire an image including at least a video. A video storage processor configured to start, when the multiple-person determiner determines that the multiple persons are on the image, storage of the video acquired by the image acquisitor to store the video in the video storage includes the first video processor of the sensor device SU and the second video processor of the management server device SV.

Next, operation of the present embodiment will be described. In the monitoring target person monitoring system MS having the above-described configuration, when power is applied, each device SU, SV, SP, TA executes initialization of each of necessary units, and starts operation of such units. By execution of the control processing programs, the SU control unit 141, the action sensing processor 142, the nurse call processor 143, the SU streaming processor 144, the multiple-person determiner 145, and the first video processor 146 are functionally configured in the SU control processor 14 of the sensor device SU. By execution of the control processing programs, the SV control unit 211, the SV monitoring processor 222, and the second video processor 223 are functionally configured in the SV control processor 21 of the management server device SV. By execution of the control processing programs, the TA control unit 321, the TA monitoring processor 322, the call processor 323, and the TA streaming processor 324 are functionally configured in the TA control processor 32 of the mobile terminal device TA.

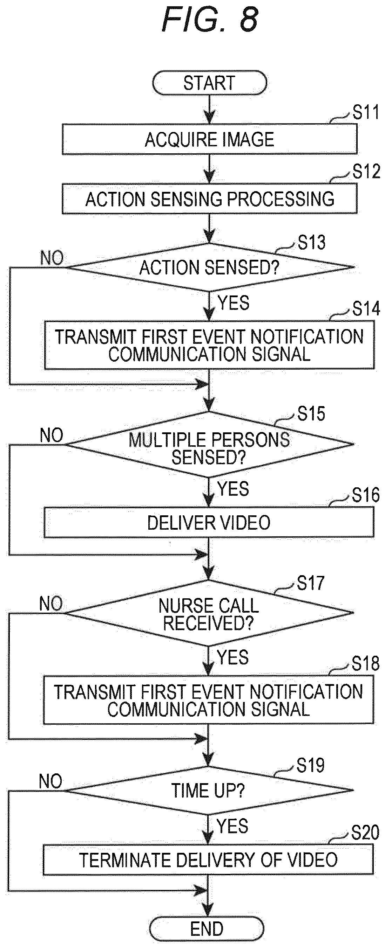

First, operation of the sensor device SU will be described. Fig. 8 is a flowchart of operation of the sensor device illustrated in Fig. 2.

The sensor device SU operates as follows in every frame or in every several frames, thereby sensing predetermined movement of the monitoring target person Ob and determining the presence or absence of the received nurse call.

In Fig. 8, the sensor device SU first acquires, as the target image, an image (image data) for a single frame from the image capturing unit 11 by the SU control unit 141 of the SU control processor 14 (S11).

Next, the sensor device SU executes, based on the target image acquired in the processing S11, the action sensing processing of sensing the predetermined action of the monitoring target person Ob by the action sensing processor 142 of the SU control processor 14 (S12). More specifically, the action sensing processor 142 determines outcome of sitting-up, outcome of leaving, outcome of falling, and outcome of overturning.

Next, the sensor device SU determines, by the action sensing processor 142, whether or not the predetermined action of the monitoring target person Ob has been sensed in the action sensing processing S12. As a result of determination, in a case where no predetermined action has been sensed (No), the sensor device SU subsequently executes processing S15. On the other hand, in a case where the predetermined action has been sensed (Yes), the sensor device SU executes the processing S15 after having executed subsequent processing S14.

In the processing S14, for notifying the predetermined action sensed in the processing S12 and the processing S13 to the predetermined terminal device SP, TA via the management server device SV, the sensor device SU transmits, as the predetermined event, the first event notification communication signal regarding sensing of the predetermined action to the management server device SV by the action sensing processor 142. More specifically, the action sensing processor 142 transmits the first event notification communication signal to the management server device SV via the SU communication IF unit 15, the first event notification communication signal containing the sensor ID of the subject unit, the predetermined event (in this embodiment, one or more of sitting-up, leaving, falling, and overturning), and the target image.

In the processing S15, the sensor device SU uses the multiple-person determiner 145 to determine, based on the target image acquired in the processing S11, whether or not the multiple persons are on the target image. As a result of determination, in a case where no multiple persons are on the target image (No), the sensor device SU subsequently executes processing S17. On the other hand, in a case where the multiple persons are on the target image (Yes), the sensor device SU executes the processing S17 after having executed subsequent processing S16.

In the processing S16, the sensor device SU functionally generates a timer in the SU control processor 14 by the first video processor 146, and sets the generated timer to a preset predetermined time (a delivery time such as five minutes, 10 minutes, or 15 minutes) to start time measurement. The sensor device SU starts delivery of the video acquired by the image capturing unit 11 to the management server device SV. More specifically, the first video processor 146 sequentially generates the video delivery communication signal containing the sensor ID of the subject unit and containing the predetermined number of frames of the video acquired by the image capturing unit 11 until the delivery ends after it has been, by each of processing S19 and processing S20 as described later, determined that the delivery time had elapsed, and transmits each of the sequentially-generated video delivery communication signals to the management server device SV.

In the processing S17, the sensor device SU determines, by the nurse call processor 143, whether or not the nurse call has been received. That is, the processing S11 to the processing S20 illustrated in Fig. 8 are repeatedly executed in every frame or in every several frames, and it is determined whether or not the nurse call receiving operation unit 13 is operated between execution of previous processing S17 and execution of current processing S17. As a result of determination, in a case where the nurse call receiving operation unit 13 is not operated and no nurse call is received (No), the sensor device SU subsequently execute the processing S19. On the other hand, in a case where the nurse call receiving operation unit 13 is operated and the nurse call has been received (Yes), the sensor device SU executes the processing S19 after having executed the subsequent processing S18.

In the processing S18, for informing the nurse call determined as received in the processing S17 to the predetermined terminal device SP, TA via the management server device SV, the sensor device SU transmits, by the nurse call processor 143, the first event notification communication signal regarding the nurse call to the management server device SV. More specifically, the nurse call processor 143 transmits the first event notification communication signal to the management server device SV via the SU communication IF unit 15, the first event notification communication signal containing the sensor ID of the subject unit and nurse call receiving information.

In the processing S19, the sensor device SU determines, by the first video processor 146, whether or not the timer functionally generated by the SU control processor 14 is up due to a lapse of the delivery time. As a result of determination, in a case where the timer is not up, the sensor device SU terminates current processing. On the other hand, in a case where the timer is up (Yes), the sensor device SU terminates current processing after having executed the subsequent processing S20.

In the processing S20, the sensor device SU generates, by the first video processor 146, the last video delivery communication signal to transmit such a signal to the management server device SV. Then, the sensor device SU terminates delivery of the video delivery communication signal. The last video delivery communication signal contains not only the sensor ID and the predetermined number of frames of the video, but also the delivery end information.

Regarding each of sensing of the predetermined action of the monitoring target person Ob and receiving of the nurse call, the sensor device SU operates as described above.

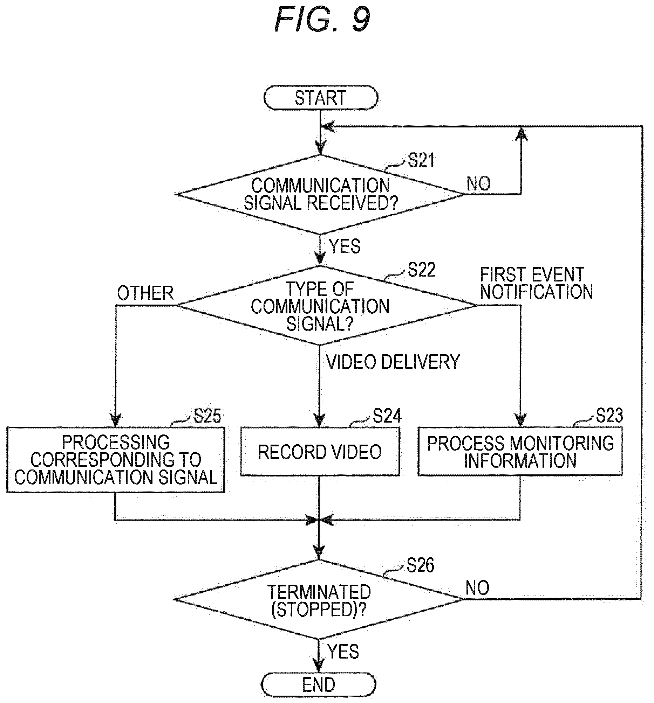

Next, operation of the management server device SV will be described. Fig. 9 is a flowchart of operation of the management server device illustrated in Fig. 3. Meanwhile, in Fig. 9, the management server device SV determines, by the SV control unit 221 of the SV control processor 22, whether or not the communication signal has been received by the SV communication IF unit 21 (S21). As a result of determination, in a case where no communication signal is received (No), the management server device SV returns the processing to S21. As a result of determination, in a case where the communication signal has been received (Yes), the management server device SV executes subsequent processing S22. That is, the management server device SV waits for receiving of the communication signal.

In the processing S22, the management server device SV determines, by the SV control unit 221, the type of received communication signal. As a result of determination, in a case where the received communication signal is the first event notification communication signal (FIRST EVENT NOTIFICATION), the management server device SV executes processing S26 after having executed processing S23. In a case where the received communication signal is the video delivery communication signal (VIDEO DELIVERY), the management server device SV executes the processing S26 after having executed processing S24. In a case where the received communication signal is neither the first event notification communication signal nor the video delivery communication signal (OTHER), the management server device SV executes the processing S26 after having executed processing S25.

In the processing S23, the management server device SV processes, by the SV monitoring processor 222 of the SV control processor 22, the first event notification communication signal received from the sensor device SU in the processing S21. More specifically, the SV monitoring processor 222 first stores (records) the monitoring information on monitoring of the monitoring target person Ob in the SV monitoring information storage 231, the monitoring information being contained in the first event notification communication signal received from the sensor device SU in the processing S21. Then, the SV monitoring processor 222 selects (searches), from the notification destination correspondence stored in the inter-device information storage 232, the notification destination corresponding to the sensor device SU having transmitted the first event notification communication signal received in the processing S21. Next, the SV monitoring processor 222 transmits the second event notification communication signal to the terminal device SP, TA as the selected notification destination. As described above, in a case where the event information contained in the first event notification communication signal received in the processing S21 is the predetermined action, the second event notification communication signal contains the sensor ID, the event information, and the target image contained in the first event notification communication signal received in the processing S21, and contains, as the video downloading destination, the communication address corresponding to the sensor device SU having the sensor ID contained in the first event notification communication signal. On the other hand, in a case where the event information contained in the first event notification communication signal received in the processing S21 is the nurse call, the second event notification communication signal contains the sensor ID and the event information contained in the first event notification communication signal received in the processing S21.

In the processing S24, the management server device SV uses the second video processor 223 of the SV control processor 22 to store the video in association with the sensor device SU (the sensor ID) in the video storage 234, the video being contained in the video delivery communication signal received from the sensor device SU in the processing S21. More specifically, when sequentially receiving a series of video delivery communication signals from the sensor device SU, the second video processor 223 brings the videos contained in the received series of video delivery communication signals into a single video file, stores the generated video file with a file name in the video storage 234, and stores, in the video storage 234, the file name of the video file in association with the sensor ID contained in the received series of video delivery communication signals. More specifically, each of the processing S21 to the processing S26 illustrated in Fig. 9 is repeatedly executed. In each repeated processing S24, the second video processor 223 stores (temporarily stores) the sensor ID and the video contained in the video delivery communication signal received in the processing S21 in association with each other in the SV storage 23. In each repeated processing S24, when the video delivery communication signal received in the processing S21 contains the delivery end information, the second video processor 223 brings a series of videos temporarily stored in the SV storage 23 into a single video file, and stores the generated video file with a file name in the video storage 234. Then, the second video processor 223 stores, in the video storage 234, the file name of the stored video file in association with the sensor ID contained in the received series of video delivery communication signals.

In the processing S25, the management server device SV executes, by the SV control processor 22, optional processing according to the communication signal received in the processing S21.

Then, in the processing S26 executed after each of the processing S23 to the processing S25, the management server device SV determines, by the SV control processor 22, whether or not operation of the management server device SV ends (stops). As a result of determination, in a case where operation ends (stops) (Yes), the management server device SV terminates the present processing. On the other hand, in a case where operation does not end (stop) (No), the management server device SV returns the processing to the processing S21.

Regarding each of the first event notification communication signal and the video delivery communication signal received from the sensor device SU, the management server device SV operates as described above.

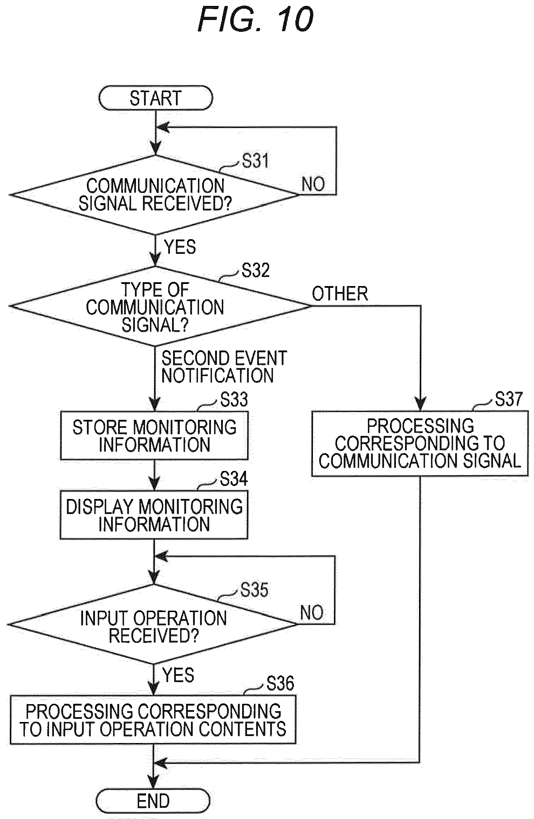

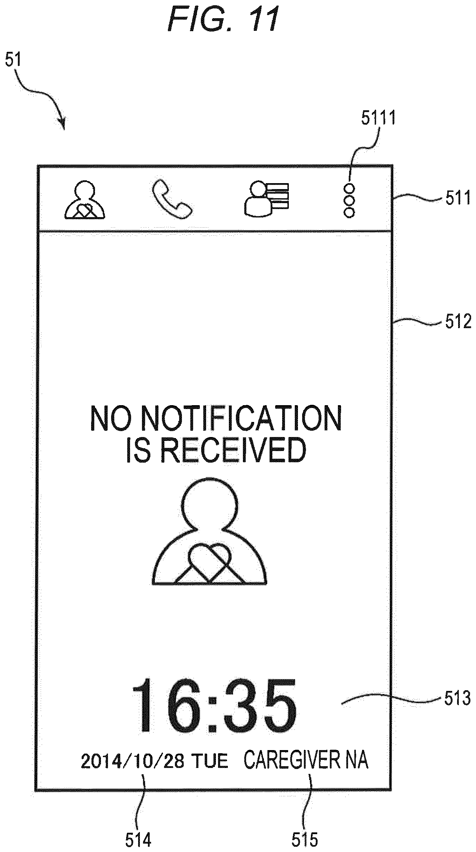

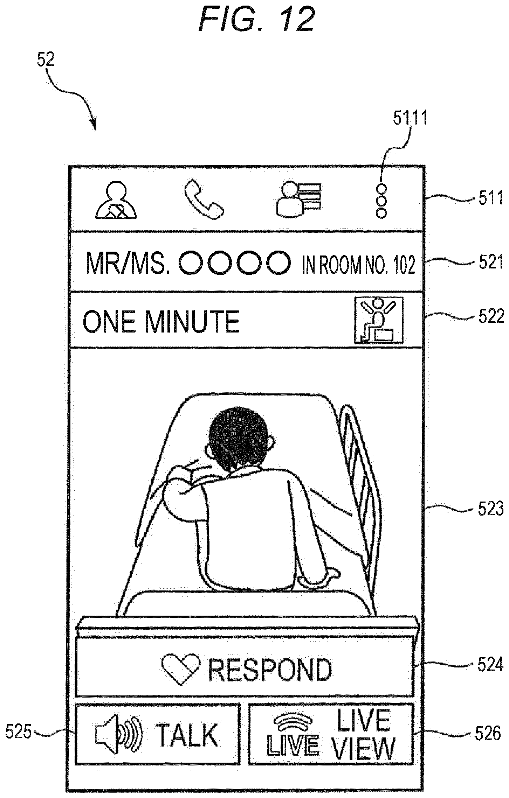

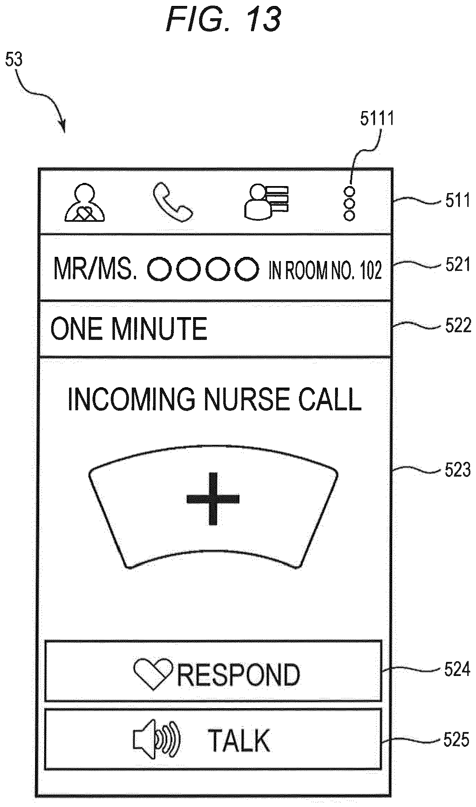

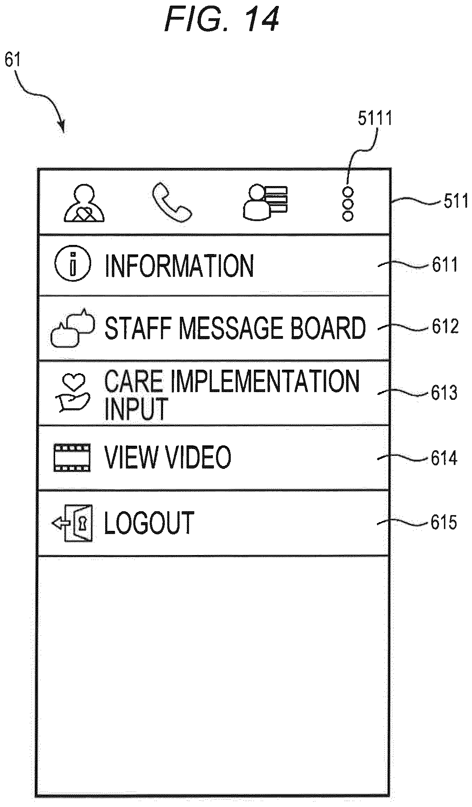

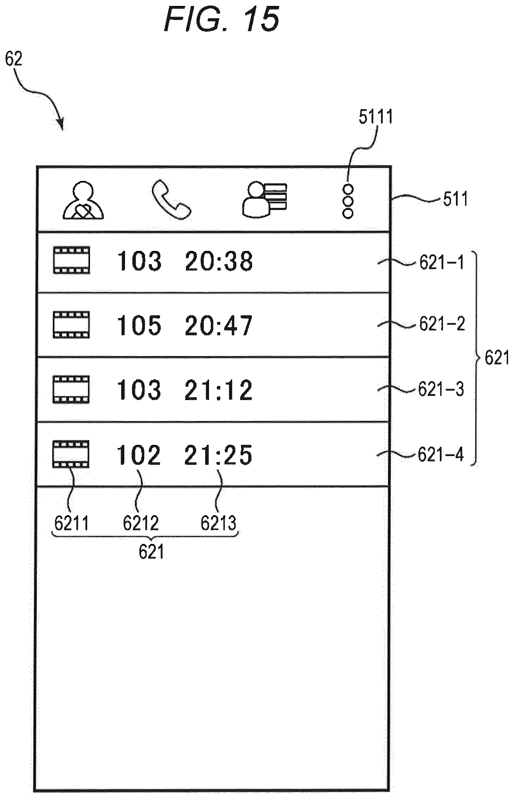

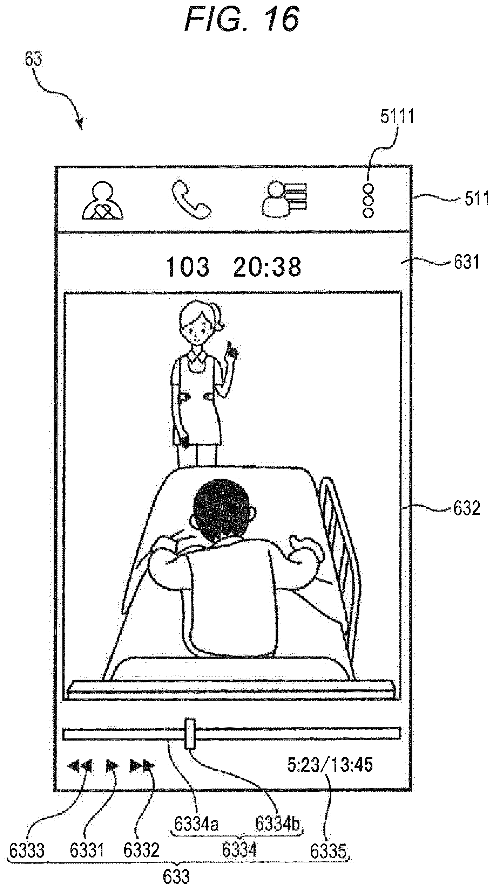

Next, operation of the terminal devices SP, TA will be described. Operation of the mobile terminal device TA will be described herein as a representative. Fig. 10 is a flowchart of operation of the mobile terminal device illustrated in Fig. 7. Fig. 11 is a view of one example of a standby screen displayed on the mobile terminal device illustrated in Fig. 7. Fig. 12 is a view of one example of a monitoring information screen displayed on the mobile terminal device illustrated in Fig. 7. Fig. 13 is a view of one example of a nurse call receiving screen displayed on the mobile terminal device illustrated in Fig. 7. Fig. 14 is a view of one example of a submenu screen displayed on the mobile terminal device illustrated in Fig. 7. Fig. 15 is a view of one example of a video selection screen displayed on the mobile terminal device illustrated in Fig. 7. Fig. 16 is a view of one example of a video display screen displayed on the mobile terminal device illustrated in Fig. 7. As described above, when power is applied to start operation, the mobile terminal device TA receives login operation by the monitoring person (the user) such as the nurse or the caregiver, and the TA monitoring processor 322 displays, on the TA display unit 36, the standby screen for waiting for the communication signal to the subject unit. As illustrated in Fig. 11, the standby screen 51 includes, for example, a menu bar region 511 for displaying a menu bar, a standby main region 512 for displaying a message (e.g., "NO NOTIFICATION IS RECEIVED") and an icon indicating a state in the middle of standby, a time region 513 for displaying a current time, a year-month-day region 514 for displaying current year, month, and day, and a user name region 515 for displaying the name of the user currently logging in the mobile terminal device TA.

In Fig. 10, the mobile terminal device TA determines, by the TA control unit 321 of the TA control processor 32, whether or not the communication signal has been received by the TA communication IF unit 31 (S31). As a result of determination, in a case where the communication signal is not received (No), the mobile terminal device TA returns the processing to S31. As a result of determination, in a case where the communication signal has been received (Yes), the mobile terminal device TA executes subsequent processing S32. That is, the mobile terminal device TA waits for receiving of the communication signal.

In the processing S32, the mobile terminal device TA determines the type of received communication signal by the TA control unit 321. As a result of determination, in a case where the communication signal received in the processing S31 is the second event notification communication signal (SECOND EVENT NOTIFICATION), the mobile terminal device TA executes subsequent processing S33. In a case where the communication signal received in the processing S31 is not the second event notification communication signal (OTHER), the present processing is terminated after the processing S37 of performing optional processing according to the communication signal received in the processing S31 has been executed.

In the processing S33, the mobile terminal device TA stores (records), by the TA monitoring processor 322 of the TA control processor 32, the monitoring information regarding monitoring of the monitoring target person Ob in the TA monitoring information storage 331, the monitoring information being contained in the second event notification communication signal received from the management server device SV in the processing S31.

Secondary to the processing S33, the TA monitoring processor 322 displays, on the TA display unit 36, the screen corresponding to each type of information contained in the second event notification communication signal received in the processing S31. More specifically, in a case where the event information contained in the second event notification communication signal received in the processing S31 is the predetermined action, the TA monitoring processor 322 displays, for example, the monitoring information screen 52 illustrated in Fig. 12 on the TA display unit 36. On the other hand, in a case where the event information contained in the second event notification communication signal received in the processing S31 is the nurse call, the nurse call receiving screen 53 illustrated in Fig. 13 is displayed on the TA display unit 36, for example.

The monitoring information screen 52 is a screen for displaying the monitoring information regarding monitoring of the monitoring target person Ob. As illustrated in Fig. 12, the monitoring information screen 52 includes, for example, the menu bar region 511, a monitoring target person name region 521 for displaying the arrangement location of the sensor device SU having the sensor ID contained in the second event notification communication signal received in the processing S31 and the name of the monitoring target person Ob monitored by the sensor device SU having the sensor ID, a sensing information display region 522 for displaying a time elapsed from the time of receiving the second event notification communication signal in the processing S31 (or the time of sensing the predetermined action) and the phenomenon information (the sensing result of the predetermined action) contained in the second event notification communication signal received in the processing S31, an image region 523 for displaying the image (i.e., the target image captured by the sensor device SU having the sensor ID) (in this embodiment, the still image) contained in the second event notification communication signal received in the processing S31, a "RESPOND" button 524, a "TALK" button 525, and a "LIVE VIEW" button 526.

For displaying the arrangement location of the sensor device SU and the name of the monitoring target person Ob in the monitoring target person name region 521, the sensor ID contained in the second event notification communication signal received in the processing S31 is used as a retrieval key to search the arrangement location of the sensor device SU and the name of the monitoring target person Ob from the TA sensor information storage 332, and the arrangement location of the sensor device SU and the name of the monitoring target person Ob are displayed. In the sensing information display region 522, the sensing result (in the present embodiment, each name of sitting-up, leaving, falling, and overturning) contained in the second event notification communication signal received in the processing S31 may be directly displayed. However, in the present embodiment, an icon symbolically representing the sensing result is displayed. Since the icon is displayed, each type of action and the icon symbolically representing such action are stored in advance in association with each other in the TA storage 33. In an example illustrated in Fig. 12, a sitting-up icon symbolically representing sitting-up is displayed in the sensing information display region 522. The "RESPOND" button 524 is, on the monitoring information screen 52, a button for inputting, to the mobile terminal device TA, intention expression information indicating the intention of the user of the mobile terminal device TA to perform a predetermined response (reply, handling) such as rescue, nursing care, elderly care, and assistance in response to the sensing result displayed on the monitoring information screen 52. The "TALK" button 525 is a button for requesting voice call, and is a button for inputting an instruction for communicably connecting the sensor device SU with the sensor ID and the mobile terminal device TA together via the network NW. The "LIVE VIEW" button 526 is a button for requesting a live video, and is a button for inputting an instruction for displaying the video captured by the sensor device SU with the sensor ID.

The nurse call receiving screen 53 is a screen for displaying receiving of the nurse call. As illustrated in Fig. 13, the nurse call receiving screen 53 includes, for example, the menu bar region 511, the monitoring target person name region 521, the sensing information display region 522, a nurse call receiving notification display region 531 for displaying a message (e.g., "INCOMING NURSE CALL") indicating receiving of the nurse call, the "RESPOND" button 524, and the "TALK" button 525. On the nurse call receiving screen 53, only the time elapsed from the time of receiving the second event notification communication signal in the processing S31 (or the time of receiving the nurse call) is displayed in the sensing information display region 522.

Note that the nurse call receiving screen 53 may further include the "LIVE VIEW" button 526.

Secondary to the processing S34, the mobile terminal device TA determines, by the TA control processor 32, whether or not input operation is received by the touch panel including the TA inputter 35 and the TA display unit 36 (S35). As a result of determination, in a case where no input operation is received (No), the mobile terminal device TA returns the processing to the processing S35. On the other hand, as a result of determination, in a case where the input operation has been received, the mobile terminal device TA executes subsequent processing S36.

In the processing S36, the mobile terminal device TA executes, by the TA control processor 32, optional processing according to the contents of the input operation, and terminates the present processing.

For example, when receiving input operation for the "RESPOND" button 524 (i.e., receiving the intention to respond), the mobile terminal device TA first provides, by the TA control processor 32, the indication of receiving of "RESPOND" to the monitoring information on the monitoring target person Ob, the monitoring information being currently displayed on the TA display unit 36. Then, the TA control processor 32 stores such information in the TA monitoring information storage 331. More specifically, in the monitoring information table MT-TA stored in the TA monitoring information storage 331 of the TA storage 33, the TA control processor 32 registers a response flag of "1" indicating receiving of the response in a response field 3316 of a record (in this embodiment, a record registering the monitoring information contained in the second event notification communication signal received in the processing S31) registering the monitoring information, which is currently displayed on the TA display unit 36, on the monitoring target person Ob. The mobile terminal device TA transmits, by the TA control processor 32, the communication signal (the response receiving notification communication signal) to the management server device SV, the communication signal containing the sensor ID corresponding to the monitoring information, which is displayed on the TA display unit 36, on the monitoring target person Ob and information (response receiving information) indicating receiving of "RESPOND." The management server device SV having received the response receiving notification communication signal transmits, by the SV control processor 22, a communication signal (a response receiving announcement communication signal) to the terminal devices SP, TA by broadcasting, the communication signal containing the sensor ID and the response receiving information contained in the received response receiving notification communication signal. In this manner, regarding the sensor ID corresponding to the monitoring information, which is displayed on the TA display unit 36, on the monitoring target person Ob, the indication of receiving of "RESPOND" is synchronized with each terminal device SP, TA.

For example, when the TA control processor 32 receives input operation for the "TALK" button 525, the mobile terminal device TA transmits, by the call processor 323, a communication signal (a call request communication signal) containing information indicating, e.g., a request of voice call to the sensor device SU monitoring the monitoring target person Ob displayed on the TA display unit 36, and is connected to the corresponding sensor device SU via the network NW so that voice call can be performed. With this configuration, voice call between the mobile terminal device TA and the sensor device SU is available. Note that when the TA control processor 32 receives input operation for a not-shown "END" button as a button for inputting an instruction for terminating voice call, the mobile terminal device TA transmits, by the call processor 323, a communication signal (a call end communication signal) containing information indicating, e.g., the request of terminating voice call to the sensor device SU monitoring the monitoring target person Ob displayed on the TA display unit 36. With this configuration, voice call between the mobile terminal device TA and the sensor device SU ends.

For example, when the TA control processor 32 receives input operation for the "LIVE VIEW" button 526, the mobile terminal device TA transmits, by the TA streaming processor 324, a communication signal (a video delivery request communication signal) containing information indicating, e.g., the request of delivering a live video to the sensor device SU monitoring the monitoring target person Ob currently displayed on the TA display unit 36. The mobile terminal device TA is connected to the corresponding sensor device SU so that the video can be downloaded via the network NW. The mobile terminal device TA receives the live video delivered from the sensor device SU, and displays the delivered video on the TA display unit 36 by streaming reproduction. On the monitoring information screen 52 on which the live video is displayed, the video is displayed in the image region 523, and a not-shown "LIVE END" button is displayed instead of the "LIVE VIEW" button 526. In this manner, the live video is displayed on the mobile terminal device TA. The not-shown "LIVE END" button is a button for requesting termination of the video, and is a button for inputting an instruction for terminating (stopping) delivery of the video captured by the sensor device SU with the sensor ID and terminating (stopping) displaying of the video. When the TA control processor 32 receives input operation for the "LIVE END" button, the mobile terminal device TA transmits, by the TA streaming processor 324, a communication signal (a video delivery end communication signal) containing information indicating, e.g., the request of terminating video delivery to the sensor device SU monitoring the monitoring target person Ob currently displayed on the TA display unit 36. Then, the mobile terminal device TA displays the still image on the TA display unit 36. In this manner, the mobile terminal device TA terminates displaying of the live video.

Regarding the second event notification communication signal received from the management server device SV, the mobile terminal device TA operates as described above.

When the user (the monitoring person) specifies the sensor device SU (the sensor ID or the monitoring target person name) and the mobile terminal device TA receives input operation for a recorded video request button, the mobile terminal device TA requests, by the TA control processor 32, the video captured by the specified sensor device SU from the management server device SV, and displays the video received from the management server device SV.

More specifically, the user (the monitoring person) performs, for example, input operation for a submenu button 5111 of the menu bar region 511 on the standby screen 51 illustrated in Fig. 11, the monitoring information screen 52 illustrated in Fig. 12, or the nurse call receiving screen 53 illustrated in Fig. 13. The submenu button 5111 is a button for inputting an instruction for displaying the submenu screen to the mobile terminal device TA. The submenu screen is a screen for displaying selectable submenus and selecting the submenu. When receiving input operation for the submenu button 5111 from the TA inputter 35 forming the touch panel, the mobile terminal device TA displays the submenu screen on the TA display unit 36.

As illustrated in Fig. 14, the submenu screen 61 includes, as the submenus, an "INFORMATION" button 611, a "STAFF MESSAGE BOARD" button 612, a "CARE IMPLEMENTATION INPUT" button 613, a "VIEW VIDEO" button 614, and a "LOGOUT" button 615, for example. The "INFORMATION" button 611 is a button for inputting, to the mobile terminal device TA, an instruction for displaying predetermined information such as a login name (a monitoring person's name) and the terminal ID. The "STAFF MESSAGE BOARD" button 612 is a button for inputting, to the mobile terminal device TA, an instruction for displaying a message registered (stored) in the management server device SV from the terminal device SP, TA by the monitoring person (the user). The "CARE IMPLEMENTATION INPUT" button 613 is a button for inputting, to the mobile terminal device TA, an instruction for recording a care record in the management server device SV from the terminal device SP, TA by the monitoring person. The "VIEW VIDEO" button 614 is a button for inputting, to the mobile terminal device TA, an instruction for specifying the sensor device SU (the sensor ID or the monitoring target person name) and requesting and displaying the video (the video file). The "LOGOUT" button 615 is a button for inputting a logout instruction to the mobile terminal device TA.

For displaying the video, the user (the monitoring person) performs input operation for the "VIEW VIDEO" button 614 on the submenu screen 61 illustrated in Fig. 14. When receiving input operation for the "VIEW VIDEO" button 614 from the TA inputter 35 forming the touch panel, the mobile terminal device TA transmits, by the TA control processor 32, a communication signal (a video selection screen request communication signal) to the management server device SV, the communication signal requesting a video selection screen configured to receive selection of the video file. The video selection screen request communication signal contains an order (an instruction, a command, a video selection screen request order) for requesting the video selection screen, the terminal ID of the subject unit, etc.

When receiving the video selection screen request communication signal from the mobile terminal device TA, the management server device SV first returns, by the processing S11, the processing S12, and the processing S25, a communication signal (a video selection screen notification communication signal) containing the video selection screen (an electronic file) indicating selectable video files to the mobile terminal device TA to receive selection of the video file. The selectable video files may be files selected within a suitable range from video files stored in the video storage 234. For example, video files stored in the video storage 234 until the receiving time of receiving the video selection screen request communication signal after a time point a predetermined time (e.g., one hour, three hours, six hours, 12 hours, 24 hours) ahead of the receiving time may be selected as the selectable video files.

When receiving the video selection screen notification communication signal from the management server device SV, the mobile terminal device TA displays the video selection screen on the TA display unit 36 by the TA control processor 32.

As illustrated in Fig. 15, the video selection screen 62 includes, for example, the menu bar region 511 and a selectable video display region 621 for displaying one or more selectable video files as a list. For example, in the present embodiment, the selectable video display region 621 includes a thumbnail display region 6211 for displaying a leading image of the video file as a thumbnail, a file name display region 6212 for displaying the file name of the video file, and a video recording time display region 6213 for displaying a video recording time of the video file. In the present embodiment, the file name of the video file includes the name of the arrangement location at which the sensor device SU having generated the video is arranged. Since the file name is configured as described above, the sensor device SU (the sensor ID or the monitoring target person name) can be specified by the file name via the sensor information. In an example illustrated in Fig. 15, the selectable video display region 621 includes four first to fourth selectable video display regions 621-1 to 621-4. Moreover, the selectable video display region 621 (each of the first to fourth selectable video display regions 621-1 to 621-4) is also a button (one example of a video selection button or the recorded video request button) for inputting, to the mobile terminal device TA, the video file selected by the monitoring person (the user).

For displaying a desired video, the user (the monitoring person) performs input operation for the selectable video display region (the video selection button) 621 for displaying the file name of the desired video file in the file name display region 6212 among one or more selectable video display regions 621 on the video selection screen 62 illustrated in Fig. 15. When receiving input operation for the selectable video display region (the video selection button) 621 from the TA inputter 35 forming the touch panel, the mobile terminal device TA transmits, by the TA control processor 32, a communication signal (a video request communication signal) requesting the selected video file to the management server device SV. The video surface request communication signal contains, for example, the file name (the arrangement location of the sensor device SU, and in other words, the sensor device SU, the sensor ID, or the monitoring target person name) displayed in the file name display region 6212 of the selectable video display region (the video selection button) 621 subjected to input operation by the user, an order (an instruction, a command, a video request order) for requesting the video file, and the terminal ID of the subject unit.

When receiving the video request communication signal from the mobile terminal device TA, the management server device SV acquires, by the processing S11, the processing S12, and the processing S25, the video file corresponding to the file name (the arrangement location of the sensor device SU, and in other words, the sensor device SU, the sensor ID, or the monitoring target person name) of the video file contained in the video request communication signal from the video storage 234, and returns a communication signal (a video notification communication signal) containing the acquired video file to the mobile terminal device TA.

When receiving the video notification communication signal from the management server device SV, the mobile terminal device TA displays, by the TA control processor 32, the video on the video display screen of the TA display unit 36. The video display screen is a screen for reproducing and displaying the video generated by the sensor device SU in the case of sensing the multiple persons by the sensor device SU.

As illustrated in Fig. 16, the video display screen 63 includes, for example, the menu bar region 511, a title region 631, a video region 632, and a reproduction control instruction input region 633. The title region 631 is a region for displaying the file name and video recording time of the video file. The video region 632 is a region for reproducing and displaying the video file. The reproduction control instruction input region 633 is a region for receiving an instruction for controlling reproduction operation (display operation) for the video displayed in the video region 632. In the present embodiment, the reproduction control instruction input region 633 includes a play/pause button 6331, a fast-forward button 6332, a rewind button 6333, a reproduction time position display region 6334, and a reproduction time display region 6335.

The play/pause button 6331 is a button for receiving a reproduction start instruction and a pause instruction for the video displayed in the video region 632. Every time the play/pause button 6331 receives input operation, a reproduction start button for receiving the reproduction start instruction and a pause button for receiving the pause instruction are alternately switched. When input operation for the play/pause button 6331 is received, if reproduction of the video is stopped, the TA control processor 32 starts, from such a stop point (including a leading point of the video), reproduction of the video displayed in the video region 632. On the other hand, if the video is being reproduced, the TA control processor 32 stops reproduction of the video displayed in the video region 632.

The fast-forward button 6332 is a button for receiving an instruction for increasing the reproduction speed of the video displayed in the video region 632. For example, the reproduction speed is a 1x speed, a 1.5x speed, a 2x speed, or a 4x speed. Every time the TA control processor 32 receives input operation for the fast-forward button 6332, the TA control processor 32 cyclically increases the reproduction speed of the video displayed in the video region 632 in the order of the 1x speed, the 1.5x speed, the 2x speed, and the 4x speed.

The rewind button 6333 is a button for receiving an instruction for increasing the backward reproduction speed (the reproduction speed in reverse reproduction) of the video displayed in the video region 632. For example, the backward reproduction speed is a 1x speed, a 1.5x speed, a 2x speed, or a 4x speed. Every time the TA control processor 32 receives input operation for the rewind button 6333, the TA control processor 32 cyclically increases the backward reproduction speed of the video displayed in the video region 632 in the order of the 1x speed, the 1.5x speed, the 2x speed, and the 4x speed.

The reproduction time position display region 6334 includes a progress bar 6334a indicating progress in reproduction of the video, and a progress position mark 6334b indicating a progress position (the temporal position of the image displayed in the video region 632 with respect to the entirety of the video) in reproduction of the video.

The reproduction time display region 6335 is a region for displaying the video recording time of the video and the reproduction time from the leading point (Reproduction Time/Video Recording Time).

By each type of operation of the mobile terminal device TA and the management server device SV, the video selected by the user is displayed on the video display screen 63 in the mobile terminal device TA.

As described above, the monitoring target person monitoring system MS, the sensor device SU and the management server device SV as one example of the monitoring target person monitoring device, and a monitoring target person monitoring method including these components in the present embodiment include the multiple-person determiner 145, the first video processor 146, and the second video processor 223. In a case where it is determined that the multiple persons are on the target image, delivery and storage of the video begins to store the video. Thus, in the monitoring target person monitoring system MS, the sensor device SU and the management server device SV as one example of the monitoring target person monitoring device, and the monitoring target person monitoring method, the video is recorded in the case of a high probability that other persons than the monitoring target person Ob are present. As described above, in the monitoring target person monitoring system MS, the sensor device SU and the management server device SV as one example of the monitoring target person monitoring device, and the monitoring target person monitoring method, the video is not constantly stored, and therefore, the privacy of the monitoring target person Ob can be taken into consideration. In a case where other persons are persons attempting to commit a theft etc., the sensor device SU functions as a so-called surveillance camera in the monitoring target person monitoring system MS, the sensor device SU and the management server device SV as one example of the monitoring target person monitoring device, and the monitoring target person monitoring method, and therefore, occurrence of the theft etc. can be reduced. In a case where other persons are monitoring persons, a state in elderly care or assistance for the monitoring target person can be recorded in the monitoring target person monitoring system MS, the sensor device SU and the management server device SV as one example of the monitoring target person monitoring device, and the monitoring target person monitoring method. Thus, in the monitoring target person monitoring system MS, the sensor device SU and the management server device SV as one example of the monitoring target person monitoring device, and the monitoring target person monitoring method, reduction in occurrence of the theft etc. and recording of the state in elderly care or assistance can be realized while the privacy of the monitoring target person is taken into consideration.

Note that in the above-described embodiment, in a case where the event is notified before the multiple-person determiner 145 determines that the multiple persons are on the target image, the video storage processor including the first and second video processors 146, 223 may start storing the video acquired by the image capturing unit 11 to store the video in the video storage 234. That is, the video storage processor starts storing the video acquired by the image capturing unit 11 to store the video in the video storage 234 after the event has been notified and before the multiple-person determiner 145 determines that the multiple persons are on the target image.

More specifically, the first video processor 146 may be the first video processor 146a configured to start delivering the video acquired by the image capturing unit 11 in a case where the event is notified before the multiple-person determiner 145 determines that the multiple persons are on the target image. That is, the first video processor 146a starts delivering the video acquired by the image capturing unit 11 after the action sensing processor 142 or the nurse call processor 143 has transmitted the first event notification communication signal to the management server device SV and before the multiple-person determiner 145 determines that the multiple persons are on the target image.

The user (the monitoring person) having received notification of the event by the terminal device SP, TA goes to the monitoring target person Ob as necessary. Thus, there is a high probability that other persons include the monitoring person. With this configuration, the state in elderly care or assistance can be suitably recorded.

Moreover, in the above-described embodiment, in a case where the sound input by the SU sound input/output unit 12 is equal to or greater than a predetermined first threshold before or after the multiple-person determiner 145 determines that the multiple persons are on the target image, the video storage processor including the first and second video processors 146, 223 may start storing the video acquired by the image capturing unit 11 to store the video in the video storage 234. That is, after the sound input by the SU sound input/output unit 12 has reached equal to or greater than the predetermined first threshold, if the multiple-person determiner 145 determines that the multiple persons are on the target image, the video storage processor starts storing the video acquired by the image capturing unit 11 to store the video in the video storage 234. Alternatively, after the multiple-person determiner 145 has determined that the multiple persons are on the target image, if the sound input by the SU sound input/output unit 12 reaches equal to or greater than the predetermined first threshold, the video storage processor starts storing the video acquired by the image capturing unit 11 to store the video in the video storage 234. Alternatively, after the sound input by the SU sound input/output unit 12 has reached equal to or greater than the predetermined first threshold after notification of the event, if the multiple-person determiner 145 determines that the multiple persons are on the target image, the video storage processor starts storing the video acquired by the image capturing unit 11 to store the video in the video storage 234. Alternatively, after the multiple-person determiner 145 has determined, after notification of the event, that the multiple persons are on the target image, if the sound input by the SU sound input/output unit 12 reaches equal to or greater than the predetermined first threshold, the video storage processor starts storing the video acquired by the image capturing unit 11 to store the video in the video storage 234.

More specifically, the first video processor 146 may be the first video processor 146b configured to start delivering the video acquired by the image capturing unit 11 in a case where the sound input by the SU sound input/output unit 12 is equal to or greater than the predetermined first threshold before or after the multiple-person determiner 145 determines that the multiple persons are on the target image. That is, the first video processor 146b starts delivering the video acquired by the image capturing unit 11 in a case where the multiple-person determiner 145 determines that the multiple persons are on the target image after the sound input by the SU sound input/output unit 12 has reached equal to or greater than the predetermined first threshold. Alternatively, the first video processor 146b starts delivering the video acquired by the image capturing unit 11 in a case where the sound input by the SU sound input/output unit 12 is equal to or greater than the predetermined first threshold after the multiple-person determiner 145 has determined that the multiple persons are on the target image. Alternatively, the first video processor 146b starts delivering the video acquired by the image capturing unit 11 in a case where the multiple-person determiner 145 determines that the multiple persons are on the target image after the sound input by the SU sound input/output unit 12 has reached, after notification of the event, equal to or greater than the predetermined first threshold. Alternatively, the first video processor 146b starts delivering the video acquired by the image capturing unit 11 in a case where the sound input by the SU sound input/output unit 12 is equal to or greater than the predetermined first threshold after the multiple-person determiner 145 has determined, after notification of the event, that the multiple persons are on the target image.

In a case where relatively-great sound occurs, occurrence of an accident or an incident is expected. Thus, according to the above-described configuration, a state in the accident or the incident can be suitably recorded.

Moreover, in the above-described embodiment, the sensor device SU as one example of the monitoring target person monitoring device further includes a window sensor 17 configured to sense opening of a window, as indicated by a dashed line in Fig. 2. In a case where the window sensor 17 senses that the window is open before or after the multiple-person determiner 145 determines that the multiple persons are on the target image, the video storage processor including the first and second video processors 146, 223 may start storing the video acquired by the image capturing unit 11 to store the video in the video storage 234. That is, in a case where the multiple-person determiner 145 determines that the multiple persons are on the target image after the window sensor 17 has sensed that the window is open, the video storage processor starts storing the video acquired by the image capturing unit 11 to store the video in the video storage 234. Alternatively, in a case where the window sensor 17 senses that the window is open after the multiple-person determiner 145 has determined that the multiple persons are on the target image, the video storage processor starts storing the video acquired by the image capturing unit 11 to store the video in the video storage 234. Alternatively, in a case where the multiple-person determiner 145 determines that the multiple persons are on the target image after the window sensor 17 has sensed, after notification of the event, that the window is open, the video storage processor starts storing the video acquired by the image capturing unit 11 to store the video in the video storage 234. Alternatively, in a case where the window sensor 17 senses that the window is open after the multiple-person determiner 145 has determined, after notification of the event, that the multiple persons are on the target image, the video storage processor starts storing the video acquired by the image capturing unit 11 to store the video in the video storage 234.

More specifically, the first video processor 146 may be the first video processor 146c configured to start delivering the video acquired by the image capturing unit 11 in a case where the window sensor senses that the window is open before or after the multiple-person determiner 145 determines that the multiple persons are on the target image. That is, the first video processor 146c starts delivering the video acquired by the image capturing unit 11 in a case where the multiple-person determiner 145 determines that the multiple persons are on the target image after the window sensor 17 has sensed that the window is open. Alternatively, the first video processor 146c starts delivering the video acquired by the image capturing unit 11 in a case where the window sensor 17 senses that the window is open after the multiple-person determiner 145 has determined that the multiple persons are on the target image. Alternatively, the first video processor 146c starts delivering the video acquired by the image capturing unit 11 in a case where the multiple-person determiner 145 determines that the multiple persons are on the target image after the window sensor 17 has sensed, after notification of the event, that the window is open. Alternatively, the first video processor 146c starts delivering the video acquired by the image capturing unit 11 in a case where the window sensor 17 senses that the window is open after the multiple-person determiner 145 has determined, after notification of the event, that the multiple persons are on the target image.

The window sensor 17 includes, for example, a magnet attached to a sliding screen of the window (a movable portion of the window) in a fixed manner, and a magnetic sensor, such as a Hall element, attached to a sash of the window (a fixed portion of the window) to face the magnet with the window being closed.

In hospitals, welfare facilities for the elderly, etc., windows are not openable/closable for preventing occurrence of accidents or incidents in many cases. Thus, in a case where these windows are opened, occurrence of the accidents or the incidents is expected. With this configuration, a state in the accidents or the incidents can be suitably recorded.

Moreover, in the above-described embodiment, the sensor device SU as one example of the monitoring target person monitoring device may further functionally include, in the SU control processor 14, a movement sensor 147 configured to sense a person movement speed or a movement acceleration, as indicated by a dashed line in Fig. 2. In a case where the movement speed or the movement acceleration sensed by the movement sensor 147 is equal to or greater than a predetermined second threshold before or after the multiple-person determiner 145 determines that the multiple persons are on the target image, the video storage processor including the first and second video processors 146, 223 may start storing the video acquired by the image capturing unit 11 to store the video in the video storage 234. That is, after the movement speed or the movement acceleration sensed by the movement sensor 147 has reached equal to or greater than the predetermined second threshold, if the multiple-person determiner 145 determines that the multiple persons are on the target image, the video storage processor starts storing the video acquired by the image capturing unit 11 to store the video in the video storage 234. Alternatively, after the multiple-person determiner 145 has determined that the multiple persons are on the target image, if the movement speed or the movement acceleration sensed by the movement sensor 147 is equal to or greater than the predetermined second threshold, the video storage processor starts storing the video acquired by the image capturing unit 11 to store the video in the video storage 234. Alternatively, after the movement speed or the movement acceleration sensed by the movement sensor 147 has reached, after notification of the event, equal to or greater than the predetermined second threshold, if the multiple-person determiner 145 determines that the multiple persons are on the target image, the video storage processor starts storing the video acquired by the image capturing unit 11 to store the video in the video storage 234. Alternatively, after the multiple-person determiner 145 has determined, after notification of the event, that the multiple persons are on the target image, if the movement speed or the movement acceleration sensed by the movement sensor 147 is equal to or greater than the predetermined second threshold, the video storage processor starts storing the video acquired by the image capturing unit 11 to store the video in the video storage 234.

More specifically, the first video processor 146 may be the first video processor 146d configured to start delivering the video acquired by the image capturing unit 11 in a case where the movement speed or the movement acceleration sensed by the movement sensor 147 is equal to or greater than the predetermined second threshold before or after the multiple-person determiner 145 determines that the multiple persons are on the target image. That is, the first video processor 146c starts delivering the video acquired by the image capturing unit 11 in a case where the multiple-person determiner 145 determines that the multiple persons are on the target image after the movement speed or the movement acceleration sensed by the movement sensor 147 has reached equal to or greater than the predetermined second threshold. Alternatively, the first video processor 146d starts delivering the video acquired by the image capturing unit 11 in a case where the movement speed or the movement acceleration sensed by the movement sensor 147 is equal to or greater than the predetermined second threshold after the multiple-person determiner 145 has determined that the multiple persons are on the target image. Alternatively, the first video processor 146d starts delivering the video acquired by the image capturing unit 11 in a case where the multiple-person determiner 145 determines that the multiple persons are on the target image after the movement speed or the movement acceleration sensed by the movement sensor 147 has reached, after notification of the event, equal to or greater than the predetermined second threshold. Alternatively, the first video processor 146d starts delivering the video acquired by the image capturing unit 11 in a case where the movement speed or the movement acceleration sensed by the movement sensor 147 is equal to or greater than the predetermined second threshold after the multiple-person determiner 145 has determined, after notification of the event, that the multiple persons are on the target image.

The movement sensor 147 extracts, from the target image acquired by the image capturing unit 11, a moving body region as a person region by, e.g., the background differencing technique or the frame differencing technique, thereby obtaining the position of the center of gravity of the extracted moving body region. The sensor device SU acquires, as described above, the target image in every frame or in every several frames. Thus, the movement sensor 147 obtains, as a movement amount, a difference between the position of the center of gravity of the moving body region (a previous gravity center position) previously obtained from the target image (a previous target image) acquired by the image capturing unit 11 and the position of the center of gravity of the moving body region (a current gravity center position) currently obtained from the target image (a current target image) acquired by the image capturing unit 11, and divides the obtained movement amount by a time difference between a previous time point and a current time point to obtain the movement speed. Alternatively, the movement sensor 147 further divides the obtained movement speed by the time difference between the previous time point and the current time point to obtain the movement acceleration.

In the case of operation with a relatively-high movement speed or a relatively-great movement acceleration, occurrence of an accident or an incident is expected. With this configuration, a state in the accident or the incident can be suitably recorded.

Moreover, in the above-described embodiment, the sensor device SU includes the first video processor 146, and the management server device SV includes the second video processor 223 and the video storage 234. However, the sensor device SU may include, instead of the first video processor 146, the video storage processor and the video storage. In this case, the sensor device SV may transmit the video according to a request from the management server device SV or the terminal device SP, TA. Alternatively, the management server device SV may include, instead of the second video processor 223, the image acquisitor, the multiple-person determiner, and the video storage processor. In this case, the SV communication IF unit 21 corresponds to another example of the image acquisitor configured to acquire the image including at least the video.

Moreover, in the above-described embodiment, the sensor device SU uses, in a case where the multiple-person determiner 145 determines that the multiple persons are on the target image, the first video processor 146 to start generating the video in the image capturing unit 11 to transmit the video to the management server device SV. After the preset predetermined time has elapsed from the point of time of starting generation of the video, the sensor device SU terminates generation of the video by the image capturing unit 11 to terminate transmission of the video to the management server device SV. However, the timing (condition) of terminating generation of the video is not limited to above, and is changeable as necessary.

For example, at the timing of determining, by the multiple-person determiner 145, that the multiple persons are not on the target image after the multiple-person determiner 145 has determined that the multiple persons are on the target image, the sensor device SU may terminate, by the first video processor 146, generation of the video by the image capturing unit 11 to terminate transmission of the video to the management server device SV. With this configuration, generation of the video is not terminated after a lapse of the predetermined time, but the video can be generated as long as the multiple persons are on the target image.

Alternatively, the sensor device SU may start, after the multiple-person determiner 145 has determined that the multiple persons are on the target image, generating the video to transmit the video to the management server device SV, for example. Further, the sensor device SU may obtain a distance among the multiple persons, and in a case where the distance falls outside a preset range, may terminate generation of the video to terminate transmission of the video to the management server device SV. More specifically, an actual length (a true length) per picture element (pixel) obtained by calculation or actual measurement considering, e.g., an optical system magnification in the image capturing unit 11 or the arrangement position of the image capturing unit 11 is obtained, and is stored in advance in the SU storage 16. Further, the multiple-person determiner 145 obtains, at predetermined time intervals, the distance among the multiple persons based on the true length per picture element, determines whether or not the obtained distance falls within the preset range, and notifies such a determination result to the first video processor 146. The above-described range is, for example, set to 1.5 m, 2 m, or 2.5 m. In a case where the multiple-person determiner 145 determines that the multiple persons are on the target image, the first video processor 146 starts generating the video on the image capturing unit 11 to transmit the video to the management server device SV. In a case where the multiple-person determiner 145 determines as falling outside the above-described range, the first video processor 146 terminates generation of the video by the image capturing unit 11 to terminate transmission of the video to the management server device SV. With this configuration, even when there are multiple persons, these persons are apart from each other only by a predetermined distance to prevent interference (e.g., one causes harm to the other), and recording is stopped to suppress the video recording time to the minimum.

Note that from this point of view, the distance among the multiple persons may be taken into consideration for the timing of starting generation of the video. That is, the sensor device SU may obtain the distance among the multiple persons after the multiple-person determiner 145 has determined that the multiple persons are on the target image. In a case where the distance falls within the preset range, the sensor device SU may start generating the video to transmit the video to the management server device SV. Thereafter, the sensor device SU may obtain the distance among the multiple persons. In a case where the distance falls outside the preset range, the sensor device SU may terminate generation of the video to terminate transmission of the video to the management server device SV. More specifically, after having determined that the multiple persons are on the target image, the multiple-person determiner 145 further obtains, for example, the distance among the multiple persons based on the true length per picture element, and determines whether or not the obtained distance falls within the preset range. In a case where the distance falls within the preset range, the first video processor 146 starts generating the video to transmit the video to the management server device SV. Then, the multiple-person determiner 145 further obtains, at the predetermined time intervals, the distance among the persons based on the true length per picture element, and determines whether or not the obtained distance falls within the preset range. Then, the multiple-person determiner 145 transmits such a determination result to the first video processor 146. In a case where the multiple-person determiner 145 determines as falling outside the range, the first video processor 146 terminates generation of the video by the image capturing unit 11 to terminate transmission of the video to the management server device SV. With this configuration, even when there are multiple persons, these persons are apart from each other only by the predetermined distance to prevent interference, and the video recording time can be reduced to the minimum.

The present specification discloses the techniques in various forms as described above, but main techniques of these techniques will be summarized below.

A monitoring target person monitoring device according to another aspect is a monitoring target person monitoring device for sensing a predetermined event regarding a monitoring target person as a monitoring target to notify the event, and includes: an image acquisitor configured to acquire an image including at least a video; a video storage configured to store the video acquired by the image acquisitor; a multiple-person determiner configured to determine, based on the image acquired by the image acquisitor, whether or not multiple persons are on the image; and a video storage processor configured to start, in a case where the multiple-person determiner determines that the multiple persons are on the image, storing the video acquired by the image acquisitor to store the video in the video storage. Preferably, the monitoring target person monitoring device includes a sensor device and a management server device communicably connected to the sensor device. The video storage processor includes a first video processor configured to start, in a case where the multiple-person determiner determines that the multiple persons are on the image, delivering the video acquired by the image acquisitor, and a second video processor configured to store the video delivered by the first video processor in the video storage. The sensor device includes the image acquisitor, the multiple-person determiner, and the first video processor. The first video processor delivers the video to the management server device, and the management server device includes the video storage and the second video processor. Preferably, in the above-described monitoring target person monitoring device, the video storage processor terminates storage of the video after a lapse of a predetermined time after the start of storage of the video. Preferably, in the above-described monitoring target person monitoring device, the first video processor terminates delivery of the video after a lapse of a predetermined time after the start of delivery of the video. Preferably, in the above-described monitoring target person monitoring device, the predetermined event is predetermined action of the monitoring target person. Preferably, in the above-described monitoring target person monitoring device, the predetermined event is a nurse call.

This monitoring target person monitoring device includes the multiple-person determiner and the video storage processor. In a case where it is determined that the multiple persons are on the image, the monitoring target person monitoring device starts storing the video to store the video. Thus, in the above-described monitoring target person monitoring device, the video is recorded in the case of a high probability that there are other persons than the monitoring target person. As described above, the above-described monitoring target person monitoring device does not constantly store the video, and therefore, can give consideration to the privacy of the monitoring target person. In a case where other persons are persons attempting to commit a theft etc., the above-described monitoring target person monitoring device functions as a so-called surveillance camera, and therefore, occurrence of the theft etc. can be reduced. In a case where other persons are monitoring persons, the above-described monitoring target person monitoring device can record a state in elderly care or assistance for the monitoring target person. Thus, in the above-described monitoring target person monitoring device, reduction in occurrence of the theft etc. and recording of the state in elderly care or assistance can be realized while the privacy of the monitoring target person is taken into consideration.

In another aspect, in the above-described monitoring target person monitoring device, the video storage processor starts storing the video acquired by the image acquisitor to store the video in the video storage in a case where the event is notified before the multiple-person determiner determines that the multiple persons are on the image.

The monitoring person having received notification of the event goes to the monitoring target person as necessary. Thus, there is a high probability that other persons include the monitoring person. Thus, the above-described monitoring target person monitoring device can suitably record the state in elderly care or assistance.

In another aspect, the above-described monitoring target person monitoring device further includes a sound inputter to which sound is input, wherein the video storage processor starts storing the video acquired by the image acquisitor to store the video in the video storage in a case where sound input by the sound inputter is equal to or greater than a predetermined first threshold before or after the multiple-person determiner determines that the multiple persons are on the image.

In a case where relatively-great sound occurs, occurrence of an accident or an incident is expected. Thus, the above-described monitoring target person monitoring device can suitably record a state in the accident or the incident.

In another aspect, the above-described monitoring target person monitoring device further includes a window sensor configured to sense opening of a window, wherein the video storage processor starts storing the video acquired by the image acquisitor to store the video in the video storage in a case where the window sensor senses that the window is open before or after the multiple-person determiner determines that the multiple persons are on the image.

In hospitals, welfare facilities for the elderly, etc., windows are not openable/closable for preventing occurrence of accidents or incidents in many cases. Thus, in a case where these windows are opened, occurrence of the accidents or the incidents is expected. Thus, the above-described monitoring target person monitoring device can suitably record a state in the accidents or the incidents.

In another aspect, the above-described monitoring target person monitoring device further includes a movement sensor configured to sense a person movement speed or movement acceleration, wherein the video storage processor starts storing the video acquired by the image acquisitor to store the video in the video storage in a case where the movement speed or the movement acceleration sensed by the movement sensor is equal to or greater than a predetermined second threshold before or after the multiple-person determiner determines that the multiple persons are on the image.

In the case of operation with a relatively-high movement speed or a relatively-great movement acceleration, occurrence of an accident or an incident is expected. Thus, the above-described monitoring target person monitoring device can suitably record a state in the accident or the incident.

A monitoring target person monitoring method according to another aspect is a monitoring target person monitoring method for sensing a predetermined event regarding a monitoring target person as a monitoring target to notify the event, and includes: an image acquiring step of acquiring an image including at least a video; a video storage step of storing the video acquired at the image acquiring step in a video storage; a multiple-person determination step of determining, based on the image acquired at the image acquiring step, whether or not multiple persons are on the image; and a video storage processing step of starting, in a case where at the multiple-person determination step, it is determined that the multiple persons are on the image, storing the video acquired at the image acquiring step to store the video in the video storage.

This monitoring target person monitoring method includes the multiple-person determination step and the video storage processing step. In a case where it is determined that the multiple persons are on the image, the monitoring target person monitoring method starts storing the video to store the video. Thus, in the above-described monitoring target person monitoring method, the video is recorded in the case of a high probability that there are other persons than the monitoring target person. As described above, the above-described monitoring target person monitoring method does not constantly store the video, and therefore, can give consideration to the privacy of the monitoring target person. In a case where other persons are persons attempting to commit a theft etc., the above-described monitoring target person monitoring method functions as a so-called surveillance camera, and therefore, occurrence of the theft etc. can be reduced. In a case where other persons are monitoring persons, the above-described monitoring target person monitoring method can record a state in elderly care or assistance for the monitoring target person. Thus, in the above-described monitoring target person monitoring method, reduction in occurrence of the theft etc. and recording of the state in elderly care or assistance can be realized while the privacy of the monitoring target person is taken into consideration.

A monitoring target person monitoring system according to another aspect includes a terminal device and a monitoring target person monitoring device communicably connected to the terminal device and configured to sense a predetermined event regarding a monitoring target person as a monitoring target to notify the event to the terminal device. The above-described monitoring target person monitoring device is any of the above-described monitoring target person monitoring devices. Preferably, in the above-described monitoring target person monitoring system, the monitoring target person monitoring device includes a sensor device and a management server device communicably connected to the sensor device. The sensor device notifies the management server device of the predetermined event regarding the monitoring target person as the monitoring target. When receiving the notification from the sensor device, the management server device re-notifies the notification to the terminal device. The video storage processor includes a first video processor configured to start, in a case where the multiple-person determiner determines that the multiple persons are on the image, delivering the video acquired by the image acquisitor, and a second video processor configured to store the video delivered by the first video processor in the video storage. The sensor device includes the image acquisitor, the multiple-person determiner, and the first video processor. The first video processor delivers the video to the management server device, and the management server device includes the video storage and the second video processor.

This monitoring target person monitoring system includes any of the above-described monitoring target person monitoring devices. Thus, reduction in occurrence of a theft etc. and recording of a state in elderly care or assistance can be realized while the privacy of the monitoring target person is taken into consideration.

This application is based on Japanese Patent Application No. 2016-32905 filed February 24, 2016, the contents of which are included in the present application.

For expressing the present invention, the present invention has been suitably and sufficiently described above through the embodiment with reference to the drawings. However, those skilled in the art shall recognize that changes and/or modifications can be easily made to the above-described embodiment. Thus, as long as changed forms or modified forms implemented by those skilled in the art are not made without departing from the scope of the claims described in the CLAIMS, it shall be interpreted that these changed or modified forms are included in the scope of the claims.

### Industrial Applicability

According to the present invention, the monitoring target person monitoring device, the monitoring target person monitoring method, and the monitoring target person monitoring system can be provided.

## Claims

1. A monitoring target person monitoring device for sensing a predetermined event regarding a monitoring target person as a monitoring target to notify the event, the device comprising:
an image acquisitor that acquires an image including at least a video;
a video storage that stores the video acquired by the image acquisitor;
a multiple-person determiner that determines, based on the image acquired by the image acquisitor, whether or not multiple persons are on the image; and
a video storage processor that starts, in a case where the multiple-person determiner determines that the multiple persons are on the image, storing the video acquired by the image acquisitor to store the video in the video storage.

2. The monitoring target person monitoring device according to claim 1, wherein
the video storage processor starts storing the video acquired by the image acquisitor to store the video in the video storage in a case where the event is notified before the multiple-person determiner determines that the multiple persons are on the image.

3. The monitoring target person monitoring device according to claim 1 or 2, further comprising:
a sound inputter to which sound is input,
wherein the video storage processor starts storing the video acquired by the image acquisitor to store the video in the video storage in a case where sound input by the sound inputter is equal to or greater than a predetermined first threshold before or after the multiple-person determiner determines that the multiple persons are on the image.

4. The monitoring target person monitoring device according to claim 1 or 2, further comprising:
a window sensor that senses opening of a window,
wherein the video storage processor starts storing the video acquired by the image acquisitor to store the video in the video storage in a case where the window sensor senses that the window is open before or after the multiple-person determiner determines that the multiple persons are on the image.

5. The monitoring target person monitoring device according to claim 1 or 2, further comprising:
a movement sensor that senses a person movement speed or movement acceleration,
wherein the video storage processor starts storing the video acquired by the image acquisitor to store the video in the video storage in a case where the movement speed or the movement acceleration sensed by the movement sensor is equal to or greater than a predetermined second threshold before or after the multiple-person determiner determines that the multiple persons are on the image.

6. A monitoring target person monitoring method for sensing a predetermined event regarding a monitoring target person as a monitoring target to notify the event, the method comprising:
an image acquiring step of acquiring an image including at least a video;
a video storage step of storing the video acquired at the image acquiring step in a video storage;
a multiple-person determination step of determining, based on the image acquired at the image acquiring step, whether or not multiple persons are on the image; and
a video storage processing step of starting, in a case where at the multiple-person determination step, it is determined that the multiple persons are on the image, storing the video acquired at the image acquiring step to store the video in the video storage.

7. A monitoring target person monitoring system comprising:
a terminal device; and
a monitoring target person monitoring device that is communicably connected to the terminal device and senses a predetermined event regarding a monitoring target person as a monitoring target to notify the event to the terminal device,
wherein the monitoring target person monitoring device is the monitoring target person monitoring device according to any one of claims 1 to 5.
